# FASCICULE DE BREVET EUROPEEN

(11) **EP 0 853 672 B1**
(45) Date de publication et mention de la délivrance du brevet: **16.02.2005**
(21) Numéro de dépôt: 96933482.0
(22) Date de dépôt: 03.10.1996
(51) Int. Cl.: C12N 15/53, C12N 15/11, C12N 15/82, C12N 9/02, A01H 5/00

(54) **SEQUENCES D'ADN CODANT POUR UNE CINNAMOYL CoA REDUCTASE, ET LEURS APPLICATIONS DANS LE DOMAINE DE LA REGULATION DES TENEURS EN LIGNINES DES PLANTES**
FÜR EINE CINNAMOYL-COA-REDUKTASE KODIERENDE DNA SEQUENZEN, UND IHRE VERWENDUNG ZUR REGULATION DES LIGNUN-GEHALTS VON PFLANZEN
DNA SEQUENCES CODING FOR A CINNAMOYL CoA REDUCTASE, AND APPLICATIONS THEREOF IN THE CONTROL OF LIGNIN CONTENTS IN PLANTS

(30) Priorité: 03.10.1995 FR 9511623
(43) Date de publication de la demande: 22.07.1998
(73) Titulaire: CENTRE NATIONAL DE LA RECHERCHE SCIENTIFIQUE (CNRS), 75016 Paris (FR); Institut National de la Recherche Agronomique (INRA), 75338 Paris Cédex 07 (FR)
(72) Inventeur: BOUDET, Alain-Michel, F-31400 Toulouse (FR); PICHON, Magalie, F-31450 Fourquevaux (FR); GRIMA-PETTENATI, Jacqueline, F-31450 Fourquevaux (FR); BECKERT, Michel, F-63800 Cournon d'Auvergne (FR); GAMAS, Pascal, F-31240 l'Union (FR); BRIAT, Jean-François, F-34980 Saint-Clément-de-Rivière (FR)
(74) Mandataire: Grosset-Fournier, Chantal Catherine
(86) Numéro de dépôt international: PCT/FR1996/001544
(87) Numéro de publication internationale: WO 1997/012982

(56) Documents cités:
- EP-A- 0 155 872
- WO-A-93/05159
- WO-A-95/27790
- THEORETICAL AND APPLIED GENETICS, vol. 87, no. 8, pages 1006-1015, XP002006034 CARRON, T.R., ET AL.: "GENETIC MODIFICATION OF CONDENSED TANNIN BIOSYNTHESIS IN LOTUS CORNICULATUS.1. HETEROLOGOUS ANTISENSE DIHYDROFLAVONOL REDUCTASE DOWN-REGULATES TANNIN ACCUMULATION IN "HAIRY ROOT" CULTURES"
- BULL. LIASON - GROUPE POLYPHENOLS, vol. 16(PT. 2), pages 295-300, XP002006035 ROBBINS, M.P., ET AL.: "MANIPULATION OF CONDENSED TANNIN BIOSYNTHESIS IN FORAGE LEGUMES"
- J. CELL. BIOCHEM. SUPPL., vol. 17A, page 26 XP002006036 CAMPBELL, M.M., ET AL.: "Hydroxycinnamoyl-coA reductase from Eucalyptus. Molecular analysis of a key control point of lignification" & KEYSTONE SYMPOSIUM ON THE EXTRACELLULAR MATRIX OF PLANTS: MOLECULAR, CELLUALR AND DEVELOPMENTAL BIOLOGY, SANT FE, NEW MEXICO, USA, JANUARY 9-15, 1993.,
- NEW PHYTOLOGIST 129 (2). 1995. 203-236., XP002006037 BOUDET A M ET AL: "Tansley review no. 80: Biochemistry and molecular biology of lignification."
- PLANT PHYSIOLOGY (ROCKVILLE) 106 (2).OCTOBRE 1994. 625-632., XP002006038 GOFFNER D ET AL: "Purification and characterization of cinnamoyl-coenzyme A:NADP oxidoreductase in Eucalyptus gunnii."
- ABSTR.PAP.AM.CHEM.SOC.;(1996) 211 MEET., PT.1, CHED274 DEN: ACSRAL ISSN: 0065-7727 1TH ACS NATIONAL MEETING, NEW ORLEANS, LA, 24-28 MARCH, 1996., XP000618488 BOUDET A M: "Genes involved in monolignol biosynthesis and their manipulation for tailoring new lignins"
- EMBL SEQUENCE DATABASE. REL. 43. ACCESSION NO. D46598. 9-MAR-1995., XP002025776 SASAKI, T., ET AL.: "Rice cDNA, partial sequence (S11367-1A)"
- EMBL SEQUENCE DATABASE. REL. 42. ACCESSION NO. T41765. 31-JAN-1995, XP002025777 NEWMAN, T., ET AL.: "10346 Arabidopsis thaliana cDNA clone 67E6T7"
- PLANT PHYSIOLOGY, vol. 96, 1991, pages 577-583, XP002025778 LAGRIMINI, L.M.: "Wound-induced deposition of polyphenols in transgenic plants overexpressing peroxidase"
- SARNI F. ET AL: 'Purification and properties of cinnamoyl-CoA reductase and cinnamyl alcohol dehydrogenase from poplar stems (Populus X euramericana)', EUR.J.BIOCHEM., vol. 139, 1994, pages 259-265

## Description

La présente invention a pour objet l'utilisation de séquences d'ADN codant pour une cinnamoyl CoA réductase (CCR) chez les plantes. ou de tout fragment de ces séquences, ou encore de toute séquence dérivée de ces dernières, ou de leurs séquences complémentaires. dans le cadre de la mise en oeuvre de procédés de régulation du taux de lignine chez les plantes.

La lignine est un polymère aromatique hétérogène complexe qui imperméabilise et renforce les parois de certaines cellules des plantes.

La lignine est formée par polymérisation de radicaux libres dérivant de monolignols tels que les alcools paracoumarylique, coniférylique et sinapylique (Higuchi, 1985, in Biosynthesis and dégradation of wood components (T. Higuchi, ed), Academic Press, Orlando, FL, pp. 141-160).

Les lignines présentent une grande variation dans leur contenu relatif en monolignols, en fonction des espèces, et des différents tissus au sein d'une même plante.

Cette variation est probablement due et contrôlée par les différentes activités et spécificités de substrats, des enzymes nécessaires à la biosynthèse des monomères de la lignine (Higuchi, 1985, susmentionné).

Au-delà de son rôle dans la structure et le développement des plantes, la lignine représente un composant majeur de la biomasse terrestre et revêt une grande importance économique et écologique (Brown, 1985, J. Appl. Biochem. 7, 371-387; Whetten and Sederoff, 1991, Forest Ecology and Management, 43, 301-316).

Sur le plan de l'exploitation de la biomasse, il convient tout d'abord de noter que la lignine est un facteur limitant de la digestibilité et du rendement nutritionnel des plantes fourragères. En effet, il est clairement démontré que la digestibilité des plantes fourragères par les ruminants, est inversement proportionnelle à la teneur en lignines de ces plantes, la nature des lignines étant également un facteur déterminant dans ce phénomène (Buxton and Roussel, 1988, Crop. Sci., **28**, 553-558; Jung and Vogel, 1986, J. Anim., Sci., **62**, 1703-1712).

Parmi les principales plantes fourragères chez lesquelles il serait intéressant de diminuer les teneurs en lignines, on peut citer: luzerne, fétuque, maïs, fourrage utilisé en ensilage...

Notons également que des teneurs en lignines élevées sont en partie responsables de la qualité limitée des tourteaux de tournesol destinés à l'alimentation du bétail, et de la diminution des capacités germinatives de certaines semences dans le domaine de l'horticulture.

On peut souligner également que la lignification intense qui se produit lors de la conservation des organes végétaux après récolte, rend rapidement impropres à la consommation, des productions telles que l'asperge, l'igname, les carottes, etc...

Par ailleurs, il convient de noter également que plus de 50 millions de tonnes de lignines sont extraites de la matière ligneuse chaque année dans le cadre de la production de la pâte à papier dans l'industrie papetière. Cette opération d'extraction nécessaire à l'obtention de la cellulose est énergétiquement coûteuse et secondairement polluante à travers les composés chimiques mis en jeu pour l'extraction et que l'on retrouve dans l'environnement (Dean and Eriksson, 1992, Holzforschung, 46, 135-147: Whetten and Sederoff, 1991, susmentionné).

Réduire les proportions en lignines (qui selon les espèces, représentent de 20 à 30 % de la matière sèche) de quelques pour cents (2 à 5%), représenterait un gain de rendement, une économie substantielle (produits chimiques) et contribuerait à l'amélioration de l'environnement (réduction des pollutions). Etant donné les échelles d'utilisation de la matière ligneuse, ces retombées auraient des répercussions extrêmement importantes. Dans ce cas, les espèces concernées pourraient être le peuplier, l'eucalyptus, l'*Acacia mangium*, le genre *Casuarina* et l'ensemble des angiospermes et gymnospermes utilisés pour la production de pâte à papier.

Il est clair que, dans les deux domaines considérés, la réduction des taux de lignines doit être modérée pour conserver à la plante (ou à l'arbre) ses caractéristiques de rigidité et son architecture normale puisque les lignines qui consolident les parois cellulaires jouent un rôle important dans le maintien du port dressé des végétaux.

Les variations naturelles dans les teneurs en lignines observées dans la nature pour une même espèce (écarts pouvant aller jusqu'à 6-8% de la masse sèche entre individus) autorisent les diminutions évoquées plus haut.

La résistance à la dégradation de la lignine, de même que les difficultés que l'on rencontre dans le cadre de son extraction, sont probablement dues à la structure complexe de ce polymère constitué de liaisons éther et carbone-carbone entre les monomères, ainsi qu'aux nombreuses liaisons chimiques existant entre la lignine et d'autres composants de la paroi cellulaire (Sarkanen and Ludwig, 1971, in Lignins: Occurrence, Formation, Structure and Reactions (K. V. Sarkanen and C.H. Kudwig eds) New York: Wiley - Interscience, pp. 1-18).

Partant des cinnamoyls-CoA, la biosynthèse des lignines chez les plantes, est effectuée de la manière suivante:

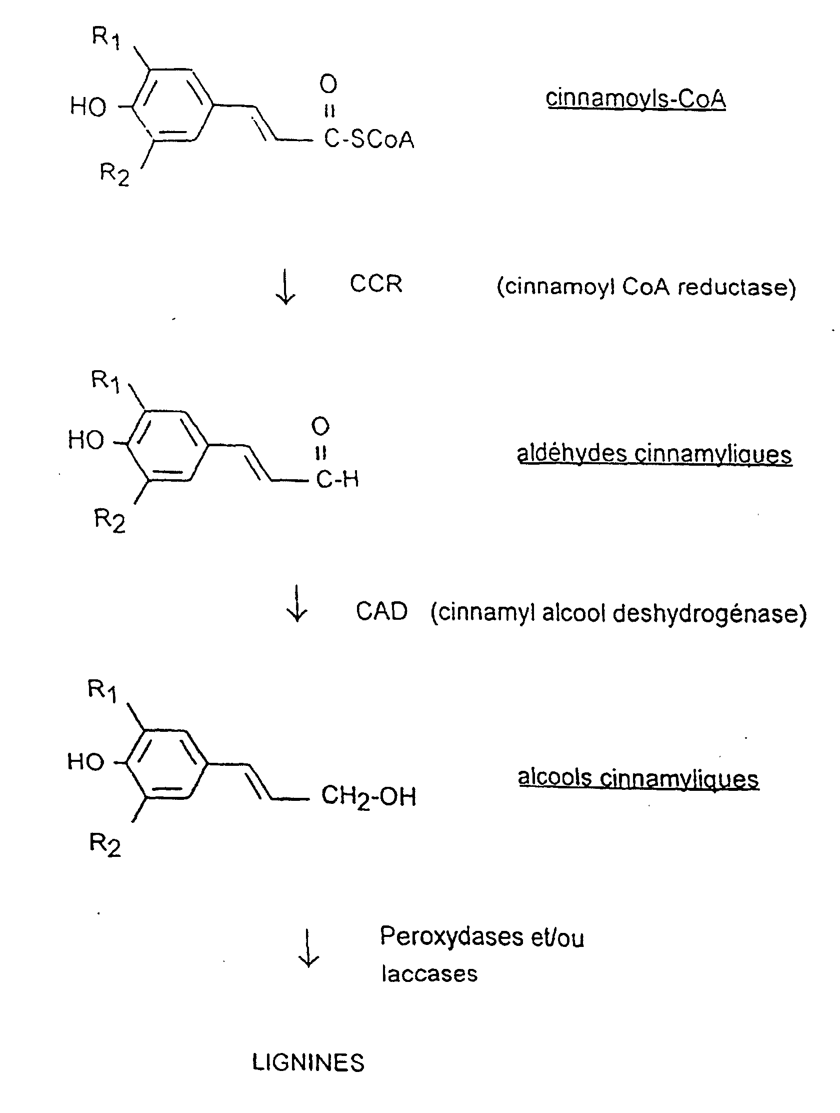

Une approche, par voie de génie génétique, pour tenter de réduire le taux de lignines chez les plantes, consisterait à inhiber la synthèse d'une des enzymes de la chaîne de la biosynthèse de ces lignines indiquées ci-dessus.

Une technique particulièrement appropriée dans le cadre d'une telle approche, est celle de l'utilisation d'ARNm antisens susceptible de s'hybrider avec l'ARNm codant pour ces enzymes, et par conséquent, d'empêcher, pour le moins partiellement, la production de ces enzymes à partir de leur ARNm correspondant.

Un telle stratégie antisens, réalisée à l'aide du gène codant pour la CAD chez le tabac, a fait l'objet de la demande de brevet européen n° 584 117, décrivant l'utilisation d'ARNm antisens susceptible d'inhiber la production de lignines chez les plantes en s'hybridant à l'ARNm codant pour la CAD chez ces plantes.

Les résultats au niveau des plantes ainsi transformées démontrent une réduction de l'activité de la CAD, mais paradoxalement, les teneurs en lignines ne montrent pas d'évolution. Des études complémentaires indiquent que les lignines de plantes transformées sont différentes des lignines témoins, car les aldéhydes cinnamyliques sont directement incorporés dans le polymère lignine.

L'un des buts de la présente invention est précisément celui de fournir un procédé permettant de réguler efficacement les teneurs en lignines dans les plantes, soit dans le sens d'une diminution sensible de ces teneurs par rapport aux teneurs normales dans les plantes, soit dans le sens d'une augmentation de ces teneurs.

Un autre but de la présente invention est de fournir les outils pour la mise en oeuvre d'un tel procédé, et plus particulièrement des constructions utilisables pour la transformation de plantes.

Un autre but de la présente invention est de fournir des plantes transformées génétiquement, notamment des plantes fourragères susceptibles d'être mieux digérées que les plantes non transformées, ou encore des plantes ou arbres transformés pour la production de la pâte à papier, et dont l'extraction des lignines serait facilitée et moins polluante que dans le cas d'arbres non transformés.

Un autre but de la présente invention est celui de fournir des plantes transformées davantage résistantes à des attaques de l'environnement, notamment à des attaques parasitaires, que ne le sont les plantes non transformées, ou encore des plantes transformées de plus grande taille, ou de taille plus réduite (que celle des plantes non transformées).

La présente invention a pour objet l'utilisation de séquences nucléotidiques recombinantes contenant une (ou plusieurs) région(s) codante(s), cette (ces) région(s) codante(s) étant constituée(s) d'une séquence nucléotidique choisie parmi les suivantes:
- la séquence nucléotidique représentée par SEQ ID NO 1, codant pour un ARNm, cet ARNm codant lui-même pour la cinnamoyl CoA réductase (CCR) de luzerne représentée par SEQ ID NO 2,
- la séquence nucléotidique représentée par SEQ ID NO 3. codant pour un ARNm, cet ARNm codant lui-même pour la CCR de maïs représentée par SEQ ID NO 4,
- un fragment de la séquence nucléotidique représentée par SEQ ID NO 1, ou de celle représentée par SEQ ID NO 3, ce fragment codant pour un fragment de la CCR représentée par SEQ ID NO 2 ou pour un fragment de la CCR représentée par SEQ ID NO 3, respectivement, ce fragment de CCR présentant une activité enzymatique équivalente à celle des deux CCR susmentionnées,
- la séquence nucléotidique complémentaire de celle représentée par SEQ ID NO 1 ou SEQ ID NO 3, cette séquence complémentaire codant pour un ARNm antisens susceptible de s'hybrider avec l'ARNm codé par les séquences SEQ ID NO 1 et SEQ ID NO 3, respectivement,
- un fragment de la séquence nucléotidique complémentaire de celle représentée par SEQ ID NO 1 ou SEQ ID NO 3, ce fragment de séquence codant pour un ARNm antisens susceptible de s'hybrider avec l'ARNm codant lui-même pour la CCR représentée par SEQ ID NO 2, ou avec l'ARNm codant lui-même pour la CCR représentée par SEQ ID NO 4, respectivement,
- la séquence nucléotidique dérivée de la séquence représentée par SEQ ID NO 1 ou SEQ ID NO 3, notamment par mutation et/ou addition, et/ou suppression, et/ou substitution d'un ou plusieurs nucléotides, cette séquence dérivée codant soit pour un ARNm codant lui-même pour la CCR représentée par SEQ ID NO 2 ou SEQ ID NO 4 respectivement, ou pour un fragment ou une protéine dérivée de ces dernières, ce fragment ou protéine dérivée présentant une activité enzymatique équivalente à celle desdites CCR chez les plantes,
- la séquence nucléotidique dérivée de la séquence nucléotidique complémentaire susmentionnée, ou du fragment de cette séquence complémentaire tel que décrit ci-dessus, par mutation et/ou addition, et/ou suppression, et/ou substitution d'un ou plusieurs nucléotides, cette séquence dérivée codant pour un ARNm antisens susceptible de s'hybrider avec un des ARNm susmentionnés,
pour la transformation de cellules végétales en vue de l'obtention de plantes transgéniques au sein desquelles la biosynthèse des lignines est régulée soit dans le sens d'une augmentation, soit dans le sens d'une diminution des teneurs en lignines produites, par rapport aux teneurs normales en lignines produites chez les plantes, et/ou dans le sens d'une modification de la composition des lignines produites par lesdites plantes transgéniques par rapport aux lignines produites chez les plantes non transformées, notamment par mise en oeuvre d'un des procédés, décrits ci-après, de régulation de la quantité de lignine chez les plantes.

Par "séquence nucléotidique dérivée", dans ce qui précède et ce qui suit, on entend toute séquence présentant au moins environ 50% (de préférence au moins 70%) de nucléotides homologues à ceux de la séquence dont elle dérive.

Par "protéine dérivée" dans ce qui précède et ce qui suit, on entend toute protéine présentant au moins environ 50% (de préférence au moins 70%) d'acides aminés homologues à ceux de la protéine dont elle dérive.

La présente invention a plus particulièrement pour objet toute séquence d'ADN, caractérisée en ce qu'elle comprend à titre de région codante:
- la séquence nucléotidique représentée par SEQ ID NO 1, codant pour un ARNm, cet ARNm codant lui-même pour la CCR représentée par SEQ ID NO 2, ou
- un fragment de la séquence nucléotidique susmentionnée, ce fragment codant pour un fragment de la CCR représentée par SEQ ID NO 2, ce fragment de CCR présentant une activité enzymatique équivalente à celle de la CCR susmentionnée, ou
- toute séquence nucléotidique dérivée de la séquence représentée par SEQ ID NO 1 susmentionnée, ou d'un fragment tel que décrit ci-dessus de cette séquence, notamment par mutation et/ou addition, et/ou suppression, et/ou substitution d'un ou plusieurs nucléotides, cette séquence dérivée codant pour un ARNm codant lui-même pour la CCR représentée par SEQ ID NO 2, ou pour une protéine dérivée de cette dernière et présentant une activité enzymatique équivalente à celle de ladite CCR chez les plantes.

La présente invention a plus particulièrement pour objet toute séquence d'ADN, caractérisée en ce qu'elle comprend à titre de région codante:
- la séquence nucléotidique représentée par SEQ ID NO 3, codant pour ARNm, cet ARNm codant lui-même pour la CCR représentée par SEQ ID NO 4, ou
- un fragment de la séquence nucléotidique susmentionnée, ce fragment codant pour un fragment de la CCR représentée par SEQ ID NO 4, ce fragment de CCR présentant une activité enzymatique équivalente à celle de la CCR susmentionnée, ou
- toute séquence nucléotidique dérivée de la séquence représentée par SEQ ID NO 3, susmentionnée, ou d'un fragment tel que décrit ci-dessus de cette séquence, notamment par mutation et/ou addition, et/ou suppression, et/ou substitution d'un ou plusieurs nucléotides, cette séquence dérivée codant pour un ARNm codant lui-même pour la CCR représentée par SEQ ID NO 4, ou pour une protéine dérivée de cette dernière et présentant une activité enzymatique équivalente à celle de ladite CCR chez les plantes.

Par protéine présentant une activité enzymatique équivalente à celle des CCR présentes chez les plantes, et plus particulièrement des CCR représentées par SEQ ID NO 2 et SEQ ID NO 4, on entend toute protéine possédant une activité CCR telle que mesurée selon la méthode de Luderitz et Grisebach publiée dans Eur. J. Biochem. (1981), **119**: 115-127.

A titre d'illustration, cette méthode est réalisée par mesure spectrophotométrique de l'activité réductrice de la protéine (CCR ou dérivée), en suivant la disparition des cinnamoyl CoA à 366 nm. La réaction se déroule à 30°C, pendant 2 à 10 minutes. La composition du milieu réactionnel est la suivante: tampon phosphate 100 mM, pH 6.25, 0,1 mM NADPH, 70 µM Feruloyl CoA, 5 à 100 µl d'extrait enzymatique dans un volume total de 500 µl.

L'invention a également pour objet toute séquence d'ADN, caractérisée en ce qu'elle comprend à titre de région codante:
- la séquence nucléotidique complémentaire de celle représentée par SEQ ID NO 1, cette séquence complémentaire codant pour un ARNm antisens susceptible de s'hybrider avec l'ARNm codant lui-même pour la CCR représentée par SEQ ID NO 2, à savoir l'ARNm codé par la séquence représentée par SEQ ID NO 1, ou codé par une séquence dérivée de cette dernière, telle que définie ci-dessus, ou
- un fragment de la séquence complémentaire susmentionnée, ce fragment de séquence codant pour un ARNm antisens susceptible de s'hybrider avec l'ARNm codant lui-même pour la CCR représentée par SEQ ID NO 2, tel que défini ci-dessus, ou
- toute séquence nucléotidique dérivée de la séquence complémentaire susmentionnée, ou du fragment de cette séquence complémentaire tel que décrit ci-dessus, notamment par mutation et/ou addition, et/ou suppression, et/ou substitution d'un ou plusieurs nucléotides, cette séquence dérivée codant pour un ARNm antisens susceptible de s'hybrider avec l'ARNm susmentionné.

La présente invention a plus particulièrement pour objet toute séquence d'ADN, caractérisée en ce qu'elle comprend à titre de région codante:
- la séquence nucléotidique complémentaire de celle représentée par SEQ ID NO 3, cette séquence complémentaire codant pour un ARNm antisens susceptible de s'hybrider avec l'ARNm codant lui-même pour la CCR représentée par SEQ ID NO 4, à savoir l'ARNm codé par la séquence représentée par SEQ ID NO 3, ou codé par une séquence dérivée de cette dernière, telle que définie ci-dessus, ou
- un fragment de la séquence complémentaire susmentionnée, ce fragment de séquence codant pour un ARNm antisens susceptible de s'hybrider avec l'ARNm codant lui-même pour la CCR représentée par SEQ ID NO 4, tel que défini ci-dessus, ou
- toute séquence nucléotidique dérivée de la séquence complémentaire susmentionnée, ou du fragment de cette séquence complémentaire tel que décrit ci-dessus, notamment par mutation et/ou addition, et/ou suppression. et/ou substitution d'un ou plusieurs nucléotides, cette séquence dérivée codant pour un ARNm antisens susceptible de s'hybrider avec l'ARNm susmentionné.

Il va de soi que les séquences représentées par SEQ ID NO 1 et SEQ ID NO 3, les séquences complémentaires, les séquences dérivées et les fragments de séquences de l'invention mentionnées ci-dessus, doivent être pris en considération comme étant représentées dans le sens 5' → 3'.

Ainsi, le premier nucléotide d'une séquence complémentaire dans le sens 5' → 3' telle que décrite ci-dessus, est le complément du dernier nucléotide de la séquence dans le sens 5' → 3' codant pour une CCR (ou fragment de CCR ou protéine dérivée), le second nucléotide de cette séquence complémentaire est le complément de l'avant-dernier nucléotide de la séquence codant pour une CCR, et ainsi de suite, jusqu'au dernier nucléotide de ladite séquence complémentaire qui est le complément du premier nucléotide de la séquence codant pour une CCR.

L'ARNm codé par la séquence complémentaire susmentionnée est tel que, lorsque cet ARNm est représenté dans le sens 5' → 3', son premier nucléotide correspond au dernier nucléotide de la séquence codant pour une CCR, et donc s'hybride avec le dernier nucléotide de l'ARNm codé par cette dernière, tandis que son dernier nucléotide correspond au premier nucléotide de la séquence codant pour une CCR, et donc s'hybride avec le premier nucléotide de l'ARNm codé par cette dernière.

Ainsi, on entend par ARNm antisens dans ce qui précède et ce qui suit, tout ARNm codé par la susdite séquence complémentaire et représenté dans le sens inverse (3' → 5') du sens dans lequel est représenté l'ARNm codé par la séquence codant pour une CCR (ou fragment de CCR ou protéine dérivée), ce dernier ARNm étant encore désigné ARNm sens (5' → 3').

Le terme d'ARN antisens s'adresse donc à une séquence d'ARN complémentaire de la séquence en bases de l'ARN messager, le terme complémentaire devant être compris en ce sens que chaque base (ou une majorité de bases) de la séquence antisens (lue dans le sens 3' → 5') est capable de s'apparier avec les bases correspondantes (G avec C, A avec U) de l'ARN messager (séquence lue dans le sens 5' → 3').

La stratégie des ARN antisens, dans le cadre de la présente invention, est une approche moléculaire particulièrement adaptée à l'objectif d'une modulation des taux de lignines des plantes. L'ARN antisens est un ARN produit par la transcription du brin d'ADN non codant (brin non-sens).

Cette stratégie antisens est plus particulièrement décrite dans le brevet européen n° 240 208.

On pense que l'inhibition de la synthèse d'une protéine selon la stratégie antisens, en l'occurrence de la CCR dans le cas présent, est la conséquence de la formation d'un duplex entre les deux ARN complémentaires (sens et antisens) empêchant ainsi la production de la protéine. Le mécanisme cependant reste obscur. Le complexe ARN-ARN peut interférer soit avec une transcription ultérieure, soit avec la maturation, le transport ou la traduction ou bien encore conduire à une dégradation de l'ARNm.

Une combinaison de ces effets est également possible.

L'invention concerne également tout ARNm codé par une séquence d'ADN selon l'invention, et plus particulièrement:
- l'ARNm codé par la séquence d'ADN représentée par SEQ ID NO 1, ou codé par un fragment ou une séquence dérivée tels que définis ci-dessus, ledit ARNm étant susceptible de coder à son tour pour la CCR présente chez la luzerne, telle que représentée par SEQ ID NO 2, ou pour un fragment de cette CCR ou une protéine dérivée tels que définis ci-dessus,
- l'ARNm codé par la séquence d'ADN représentée par SEQ ID NO 3, ou codé par un fragment ou une séquence dérivée tels que définis ci-dessus, ledit ARNm étant susceptible de coder à son tour pour la CCR présente chez le maïs, telle que représentée par SEQ ID NO 4, ou pour un fragment de cette CCR ou une protéine dérivée tels que définis ci-dessus.

L'invention a également pour objet tout ARNm antisens, tel que défini ci-dessus, caractérisé en ce qu'il comprend des nucléotides complémentaires de la totalité ou d'une partie seulement des nucléotides constituant un ARNm tel que décrit ci-dessus selon l'invention, ledit ARNm antisens étant susceptible de s'hybrider (ou de s'apparier) avec ce dernier.

A ce titre, l'invention vise plus particulièrement les ARNm antisens codés par des séquences d'ADN selon l'invention, comprenant au moins une région de 50 bases homologues à celles d'une région des séquences complémentaires des séquences d'ADN susmentionnées de l'invention.

Il n'y a pas de limite supérieure de taille pour les séquences d'ADN codant pour un ARN antisens selon l'invention; elles peuvent être aussi longues que celles du messager normalement produit dans les cellules, voire aussi longues que la séquence d'ADN génomique codant pour l'ARNm de la CCR.

Avantageusement, de telles séquences d'ADN codant pour un ARN antisens selon l'invention, comprennent entre environ 100 et environ 1000 paires de bases.

L'invention a plus particulièrement pour objet toute séquence antisens comprenant un (ou plusieurs) ARNm antisens tel(s) que décrit(s) ci-dessus, ou fragment(s) de cet (ces) ARNm antisens, et une (ou plusieurs) séquence(s) correspondant à un (ou plusieurs) domaine(s) catalytique(s) d'un ribozyme.

A ce titre, l'invention vise plus particulièrement toute séquence antisens telle que décrite ci-dessus, comprenant le domaine catalytique d'un ribozyme flanqué de part et d'autre par des bras d'environ 8 bases complémentaires des séquences bordant un motif GUX (X représentant C, U ou A) comprises dans l'un des ARNm de l'invention décrits ci-dessus (encore désignés ARN cibles) (Haseloff J., et Gerlach W. L., 1988, Nature, **334**: 585-591).

L'invention concerne également toute séquence d'ADN susceptible de coder pour une séquence antisens telle que décrite ci-dessus comprenant au moins un domaine catalytique d'un ribozyme lié à un ou plusieurs ARNm antisens de l'invention, ou fragment(s) d'ARNm antisens (avantageusement des fragments d'environ 8 bases tels que décrits ci-dessus).

L'invention a plus particulièrement pour objet:
- tout ARNm antisens, tel que décrit ci-dessus, caractérisé en ce qu'il est codé par la séquence nucléotidique complémentaire de celle représentée par SEQ ID NO 1, ledit ARNm antisens étant susceptible de s'hybrider avec l'ARNm codé par la séquence d'ADN représentée par SEQ ID NO 1,
- tout ARNm antisens, tel que décrit ci-dessus, caractérisé en ce qu'il est codé par la séquence nucléotidique complémentaire de celle représentée par SEQ ID NO 3, ledit ARNm antisens étant susceptible de s'hybrider avec l'ARNm codé par la séquence d'ADN représentée par SEQ ID NO 3.

L'invention concerne également les polypeptides recombinants codés par les séquences d'ADN de l'invention, lesdits polypeptides recombinants présentant une activité enzymatique équivalente à celle des CCR chez les plantes, et plus particulièrement les CCR recombinantes codées par les séquences représentées par SEQ ID NO 1 et SEQ ID NO 3, ou par des séquences dérivées de ces dernières selon l'invention.

L'invention a plus particulièrement pour objet les polypeptides recombinants, et notamment les CCR recombinantes, tels qu'obtenus par transformation de cellules végétales en intégrant de façon stable dans leur génome, une séquence nucléotidique recombinante telle que définie ci-après, contenant une séquence d'ADN selon l'invention, notamment à l'aide d'un vecteur tel que décrit ci-après.

Par l'expression "polypeptides recombinants", on doit entendre toute molécule possédant une chaîne polypeptidique susceptible d'être produite par génie génétique, par l'intermédiaire d'une phase de transcription de l'ADN du gène correspondant, ce qui conduit à l'obtention d'ARN qui est par la suite transformé en ARNm (par suppression des introns), ce dernier étant ensuite traduit par les ribosomes, sous forme de protéines, le tout étant effectué sous contrôle d'éléments de régulation appropriés à l'intérieur d'une cellule hôte. Par conséquent, l'expression "polypeptides recombinants" utilisée n'exclut pas la possibilité que lesdits polypeptides comprennent d'autres groupements, tels que les groupements glycosylés.

Bien entendu, le terme "recombinant" indique que le polypeptide a été produit par génie génétique, car il résulte de l'expression, dans un hôte cellulaire approprié, de la séquence nucléotidique correspondante qui a été auparavant introduite dans un vecteur d'expression utilisé pour transformer ledit hôte cellulaire. Toutefois, ce terme "recombinant" n'exclut pas la possibilité que le polypeptide soit produit par un procédé différent, par exemple par synthèse chimique classique selon les méthodes connues utilisées pour la synthèse de protéines, ou par clivage protéolytique de molécules de plus grande taille.

L'invention concerne plus particulièrement la CCR telle que présente dans les cellules de luzerne et représentée par SEQ ID N0 2 ou la CCR telle que présente dans les cellules de maïs et représentée par SEQ ID NO 4, lesdites CCR étant telles qu'obtenues sous forme essentiellement pure par extraction et purification à partir de luzerne ou de maïs, ou toute protéine dérivée de ces dernières, notamment par addition, et/ou suppression, et/ou substitution d'un ou plusieurs acides aminés, ou tout fragment issu desdites CCR ou de leurs séquences dérivées, lesdits fragments et séquences dérivées étant susceptibles de posséder une activité enzymatique équivalente à celle des CCR susmentionnées.

L'invention a également pour objet les séquences nucléotidiques codant pour la CCR représentée par SEQ ID NO 2 ou SEQ ID NO 4, ou toute séquence dérivée ou fragment de ces dernières, tels que définis ci-dessus, lesdites séquences nucléotidiques étant caractérisées en ce qu'elles correspondent à tout ou partie des séquences représentées par SEQ ID NO 1 ou SEQ ID NO 3 respectivement, ou à toute séquence dérivée de ces dernières par dégénérescence du code génétique, et étant néanmoins susceptibles de coder pour les CCR ou séquence dérivée ou fragment de ces dernières, tels que définis ci-dessus.

L'invention vise également les complexes formés entre les ARNm antisens, tels que décrits ci-dessus, et les ARNm selon l'invention, susceptibles de coder pour tout ou partie d'une CCR chez les plantes.

L'invention a plus particulièrement pour objet le complexe formé entre l'ARNm codé par la séquence SEQ ID NO 1 et l'ARNm antisens codé par la séquence complémentaire de la séquence SEQ ID NO 1. ainsi que le complexe formé entre l'ARNm codé par la séquence SEQ ID NO 3 et l'ARNm antisens codé par la séquence complémentaire de la séquence SEQ ID NO 3.

L'invention a plus particulièrement pour objet toute séquence nucléotidique recombinante (ou ADN recombinant), caractérisée en ce qu'elle comprend au moins une séquence d'ADN selon l'invention, choisie parmi celles décrites ci-dessus, ladite séquence d'ADN étant insérée dans une séquence hétérologue.

L'invention concerne plus particulièrement toute séquence nucléotidique recombinante telle que décrite ci-dessus, comprenant, à titre de région codante, la séquence nucléotidique représentée par SEQ ID NO 1, ou par SEQ ID NO 3, ou tout fragment ou une séquence nucléotidique dérivée de ces dernières, tels que définis ci-dessus, lesdites séquences nucléotidiques ou ledit fragment étant insérés dans une séquence hétérologue, et étant susceptibles de coder pour la CCR représentée par SEQ ID NO 2, ou par SEQ ID NO 4 respectivement, ou pour un fragment de ces CCR, ou pour une protéine dérivée de ces dernières, tels que définis ci-dessus.

L'invention concerne plus particulièrement encore, toute séquence nucléotidique recombinante comprenant, à titre de région codante, une séquence nucléotidique complémentaire de celle représentée par SEQ ID NO 1, ou par SEQ ID NO 3, ou tout fragment ou toute séquence nucléotidique dérivée de cette séquence complémentaire, tels que définis ci-dessus, lesdites séquences complémentaires ou ledit fragment étant insérés dans une séquence hétérologue, et étant susceptibles de coder pour un ARNm antisens capable de s'hybrider avec tout ou partie de l'ARNm codant pour une CCR chez les plantes, et plus particulièrement avec tout ou partie de l'ARNm codant pour la CCR représentée par SEQ ID NO 2, ou par SEQ ID NO 4.

Les ADN recombinants selon l'invention sont davantage caractérisés en ce qu'ils comprennent les éléments nécessaires pour réguler l'expression de la séquence nucléotidique codant pour une CCR, ou de sa séquence complémentaire codant pour un ARNm antisens selon l'invention, notamment un promoteur et un terminateur de la transcription de ces séquences.

Parmi les différents promoteurs susceptibles d'être utilisés dans les constructions d'ADN recombinants selon l'invention, on peut citer:
- le promoteur endogène contrôlant l'expression de la CCR chez une plante, notamment le promoteur situé en amont de la séquence d'ADN représentée par SEQ ID NO 5 codant chez l'eucalyptus pour la CCR représentée par SEQ ID NO 6, ou
- des promoteurs de type constitutif à forte expression, exemples: ³⁵S CAMV (décrit dans Benfey et al. (1990), EMBO J., **9** (6), 1677-1684), EF1α (promoteur du gène d'un facteur d'élongation dans la synthèse protéique décrit par Curie et al. (1991), Nucl. Acids Res., **19**, 1305-1310),
- des promoteurs de type spécifique à expression particulière dans des tissus individuels, exemples: promoteur CAD (décrit par Feuillet C. (1993), Thèse de l'Université de Toulouse III), promoteur GRP 1-8 (décrit par Keller et Baumgartner, (1991), Plant Cell., 3, 1051-1061) à expression dans des tissus vasculaires spécifiques.

L'invention concerne également toute séquence nucléotidique recombinante telle que décrite ci-dessus, et comprenant également à titre de région codante au moins une séquence nucléotidique codant pour tout ou partie d'un ARNm codant lui-même pour une autre enzyme que la CCR, qui se trouve être impliquée dans une étape de la biosynthèse des lignines chez les plantes, notamment l'ARNm codant pour l'alcool cinnamylique deshydrogénase (CAD), ou comprenant également à titre de région codante au moins une séquence nucléotidique codant pour tout ou partie d'un ARNm antisens susceptible de s'hybrider avec l'ARNm susmentionné, notamment avec l'ARNm codant pour la CAD.

Les séquences nucléotidiques recombinantes susmentionnées de l'invention sont avantageusement obtenues à partir de vecteurs dans lesquels sont insérées les séquences d'ADN codant pour une enzyme nécessaire à la biosynthèse des lignines chez les plantes.

Les vecteurs susmentionnés sont digérés à l'aide d'enzymes de restriction appropriées afin de récupérer lesdites séquences d'ADN qui s'y trouvent insérées.

Ces dernières sont ensuite insérées en aval d'un promoteur approprié, et en amont d'un terminateur approprié de l'expression, au sein des ADN recombinants selon l'invention.

L'invention vise plus particulièrement les ADN recombinants comprenant la séquence représentée par SEQ ID NO 1 ou celle représentée par SEQ ID NO 3, tels qu'obtenus par digestion de vecteurs susmentionnés, récupération de la séquence d'ADN de l'invention et insertion de cette dernière dans le sens 5' → 3', au sein d'une séquence d'ADN hétérologue comprenant un promoteur et un terminateur de l'expression de ladite séquence.

L'invention a également plus particulièrement pour objet les ADN recombinants comprenant la séquence complémentaire de la séquence représentée par SEQ ID NO 1, ou de celle représentée par SEQ ID NO 3, tels qu'obtenus par digestion de vecteurs susmentionnés, récupération de la séquence d'ADN de l'invention, et insertion de cette dernière en sens inverse, c'est-à-dire dans le sens 3' → 5', au sein d'une séquence d'ADN hétérologue comprenant un promoteur et un terminateur de l'expression de la séquence complémentaire.

A titre d'exemple de terminateur utilisable dans de telles constructions, on peut citer l'extrémité 3' du gène de la nopaline synthase d'*Agrobacterium tumefaciens*.

Ainsi, d'une manière générale, les séquences nucléotidiques recombinantes selon l'invention, contenant une séquence d'ADN codant pour une CCR (ou fragment de CCR ou protéine dérivée), et/ou d'autres enzymes nécessaires à la biosynthèse des lignines, sont obtenues par récupération de ladite séquence d'ADN à partir des vecteurs susmentionnés, et insertion de cette séquence dans la séquence hétérologue, tandis que les séquences nucléotidiques recombinantes contenant une séquence d'ADN codant pour un ARNm antisens selon l'invention, sont obtenus par récupération de la séquence d'ADN susmentionnée et insertion en sens inverse de cette dernière dans ladite séquence hétérologue.

A titre d'illustration, on peut utiliser tout ou partie de l'ADN complémentaire (ADNc) représenté par SEQ ID NO 1 ou SEQ ID NO 3, pour la construction des ADN recombinants susmentionnés, ou bien tout ou partie du clone génomique correspondant à une CCR (qui correspond aux ADNc susmentionnés + d'éventuels introns). Ce clone génomique peut être obtenu en utilisant les ADNc comme sondes pour cribler une banque génomique, cette dernière étant elle-même obtenue suivant la méthode décrite par Sambrook, Fritsch et Maniatis, Molecular Cloning Laboratory Manual, Cold Spring Harbour Laboratory Press, 1989.

L'invention a également pour objet tout vecteur recombinant, utilisable pour la transformation de plantes, caractérisé en ce qu'il comprend une séquence nucléotidique recombinante choisie parmi celles décrites ci-dessus, selon l'invention, intégrée dans l'un des sites de son génome non essentiels pour sa réplication.

Parmi les vecteurs recombinants susmentionnés utilisables pour la transformation de plantes, on peut citer: les vecteurs binaires dérivés de pBIN 19 (Bevan et al., (1984), Nucl. Acids Res., **12** (22), 8711-8721).

Des exemples de construction de vecteurs recombinants selon l'invention sont décrits dans la description détaillée qui suit de l'invention.

La présente invention a également pour objet un procédé de régulation de la biosynthèse des lignines chez les plantes, soit par diminution, soit par augmentation des quantités de lignines produites, par rapport aux quantités normales de lignines produites chez ces plantes, ledit procédé comprenant une étape de transformation de cellules de ces plantes à l'aide d'un vecteur contenant:
- la séquence nucléotidique représentée par SEQ ID NO 1 ou par SEQ ID NO 3 ou un fragment des séquences nucléotidiques susmentionnées, ce fragment codant pour un ARNm, cet ARNm codant lui-même pour un fragment d'une CCR chez les plantes, ce fragment de CCR présentant une activité enzymatique équivalente à celle de la CCR représentée par SEQ ID NO 2 ou par SEQ ID NO 4, ou d'une séquence nucléotidique dérivée des séquences nucléotidiques susmentionnées, ou dérivée du fragment susmentionné, notamment par mutation et/ou addition, et/ou suppression, et/ou substitution d'un ou plusieurs nucléotides, cette séquence dérivée codant pour un ARNm, cet ARNm codant lui-même pour une protéine dérivée présentant une activité enzymatique équivalente à celle de l'une au moins des CCR susmentionnées, ou
- une séquence nucléotidique complémentaire de tout ou partie des séquences nucléotidiques représentées par SEQ ID NO 1 ou par SEQ ID NO 3 codant pour un ARNm, ou du fragment de ces séquences, ou de la séquence dérivée de ces derniers, tels que définis ci-dessus, cette séquence complémentaire codant pour un ARNm antisens susceptible de s'hybrider avec l'un des ARNm susmentionnés,
ladite transformation étant effectuée notamment à l'aide d'un vecteur tel que décrit ci-dessus.

L'invention a plus particulièrement pour objet un procédé de diminution de la quantité de lignines produites par biosynthèse chez les plantes, ce procédé étant effectué par transformation du génome de ces plantes, en y incorporant:
- au moins une séquence d'ADN selon l'invention telle que décrite ci-dessus, codant pour un ARNm antisens susceptible de s'hybrider avec tout ou partie de l'ARNm codant pour la CCR représentée par SEQ ID NO 2 ou SEQ ID NO 4. ou pour une protéine dérivée de ces dernières telle que définie ci-dessus,
- et, le cas échéant, au moins une séquence d'ADN codant pour un ARNm antisens capable de s'hybrider à un ARNm codant pour une autre enzyme que la CCR, qui se trouve être impliquée dans une étape de la biosynthèse des lignines chez les plantes, notamment l'ARNm codant pour la CAD,
ladite transformation étant réalisée:
- soit à l'aide d'un vecteur recombinant tel que décrit ci-dessus, contenant une séquence d'ADN codant pour un ARNm antisens capable de s'hybrider avec l'ARNm codant pour la CCR ou pour une protéine dérivée, telle que définie ci-dessus, et, le cas échéant, contenant une ou plusieurs séquence(s) d'ADN codant pour un ARNm antisens capable de s'hybrider à un ARNm codant pour une autre enzyme que la CCR telle que définie ci-dessus,
- soit à l'aide de plusieurs vecteurs recombinants dont l'un au moins contient une séquence d'ADN codant pour un ARNm antisens capable de s'hybrider avec l'ARNm codant pour la CCR ou pour une protéine dérivée, telle que définie ci-dessus, tandis que l' (ou les) autre(s) vecteur(s) recombinant(s) contien(nen)t une séquence d'ADN codant pour un ARNm antisens capable de s'hybrider à un ARNm codant pour une autre enzyme que la CCR, telle que définie ci-dessus.

Un autre procédé de diminution de la quantité de lignines produites par biosynthèse chez les plantes, est celui réalisé par transformation du génome de ces plantes, en y incorporant:
- au moins une séquence d'ADN selon l'invention représentée par SEQ ID NO 1 ou SEQ ID NO 3, ou un fragment ou une séquence dérivée de cette dernière, tels que définis ci-dessus,
- et, le cas échéant, au moins une séquence d'ADN codant pour tout ou partie d'une autre enzyme que la CCR, qui se trouve être impliquée dans une étape de la biosynthèse des lignines chez les plantes, notamment une séquence d'ADN codant pour tout ou partie de la CAD,
ladite transformation étant réalisée:
- soit à l'aide d'un vecteur recombinant tel que décrit ci-dessus, contenant la séquence d'ADN selon l'invention susmentionnée, ou un fragment ou une séquence dérivée de cette dernière, tels que définis ci-dessus, et, le cas échéant, contenant une ou plusieurs séquence(s) d'ADN codant pour tout ou partie d'une enzyme autre que la CCR, telle que définie ci-dessus.
- soit à l'aide de plusieurs vecteurs recombinants dont l'un au moins contient une séquence d'ADN selon l'invention susmentionnée, ou un fragment ou une séquence dérivée de cette dernière, tels que définis ci-dessus, tandis que l' (ou les) autre(s) vecteur(s) recombinant(s) contien(nen)t une séquence d'ADN codant pour tout ou partie d'une enzyme autre que la CCR, telle que définie ci-dessus.

Cette dernière méthode fait appel au mécanisme de co-suppression. La co-suppression a été observée quand des copies du gène endogène ont été introduites dans le génome. Bien que le mécanisme de la co-suppression soit actuellement inconnu, une des hypothèses les plus fréquemment retenue est que la régulation négative de l'expression du gène viendrait de la production d'une faible proportion d'ARN antisens dérivée d'un transgène à travers une lecture du "mauvais" brin du transgène (Grierson et al., Trends Biotech., **9**: 122-123).

L'invention a également pour objet un procédé de diminution de la quantité de lignines produites par biosynthèse chez les plantes, ce procédé étant réalisé par transformation du génome de ces plantes en y incorporant une séquence d'ADN telle que décrite ci-dessus selon l'invention, codant pour une séquence antisens comprenant un (ou plusieurs) domaine(s) catalytique(s) d'un ribozyme lié(s) à un (ou plusieurs) ARNm antisens, ou fragment(s) d'ARNm antisens de l'invention, ladite transformation étant réalisée à l'aide d'un vecteur recombinant comprenant une séquence nucléotidique recombinante selon l'invention contenant elle-même la séquence d'ADN susmentionnée.

Il est important de noter que les méthodes susmentionnées permettent d'aboutir à des plantes transformées présentant des niveaux différents de réduction de l'activité CCR (selon le niveau d'insertion de la séquence d'ADN codant pour l'ARNm antisens, le nombre de copies de cette séquence d'ADN intégrée dans le génome...), et donc des teneurs en lignines.

Le choix des transformants permettra donc une modulation contrôlée des teneurs en lignines compatible avec un développement normal de la plante.

D'une manière générale, si l'on considère que la teneur moyenne normale en lignines d'une plante varie entre environ 15% et environ 35% en poids de matière sèche, la réduction de la teneur en lignines résultant de la mise en oeuvre d'un des procédés susmentionnés, est avantageusement telle que les plantes ainsi transformées présentent une teneur moyenne en lignines variant entre environ 10% et environ 30%, ou encore entre environ 12% et environ 32%.

A titre d'illustration, la teneur en lignines d'une plante peut être mesurée selon une variante de la méthode de Johnson et al., (1961), T.A.P.P.I., **44**, 793-798, qui est décrite en détail dans Alibert et Boudet (1979), Physiol., Veg., **17** (1), 67-74, et dont les principales étapes sont les suivantes: après obtention d'une poudre alcool benzène contenant les lignines du matériel végétal, les lignines sont solubilisées par le bromure d'acétyle et dosées en fonction de leur absorption dans l'ultraviolet.

L'invention vise plus particulièrement l'application des procédés susmentionnés de diminution des teneurs en lignines dans les plantes, à l'obtention de plantes fourragères transformées génétiquement, présentant des teneurs en lignines réduites par rapport aux teneurs normales en lignines chez ces plantes, et dont la digestibilité se trouve être ainsi améliorée par rapport à ces mêmes plantes non transformées.

Parmi les principales plantes fourragères susceptibles d'être transformées dans le cadre de la présente invention, on peut citer: la luzerne, la fétuque, le maïs destiné à l'ensilage, etc...

L'invention concerne également l'application des procédés susmentionnés de diminution des teneurs en lignines chez les plantes, à l'obtention de plantes, et plus particulièrement d'arbres, transformés génétiquement, présentant des teneurs en lignines réduites par rapport aux teneurs normales en lignines chez ces plantes, ces plantes ou arbres étant particulièrement avantageux à utiliser dans le cadre de la production de la pâte à papier.

Un troisième domaine potentiel d'application des procédés susmentionnés de régulation négative de l'expression du gène de la CCR, concerne la stimulation de la croissance des plantes transformées. Divers arguments soulignent (Sauter et Kende, 1992, Plant and Cell Physiology, **33** (8):1089), qu'une lignification précoce et rapide est une frein au grandissement cellulaire et donc à la croissance des végétaux. Ainsi la mise en oeuvre des procédés susmentionnés est susceptible de permettre pour les plantes ainsi transformées à lignification réduite une meilleure croissance et donc de meilleurs rendements.

L'invention concerne également un procédé d'augmentation de la quantité de lignines produites par biosynthèse chez les plantes, ce procédé étant effectué par transformation du génome de ces plantes, en y incorporant:
- au moins une séquence d'ADN selon l'invention représentée par SEQ ID NO 1 ou SEQ ID NO 3. ou un fragment ou une séquence dérivée de cette dernière, tels que définis ci-dessus,
- et, le cas échéant, au moins une séquence d'ADN codant pour tout ou partie d'une autre enzyme que la CCR, qui se trouve être impliquée dans une étape de la biosynthèse des lignines chez les plantes, notamment une séquence d'ADN codant pour tout ou partie de la CAD,
ladite transformation étant réalisée:
- soit à l'aide d'un vecteur recombinant tel que décrit ci-dessus, contenant la séquence d'ADN selon l'invention susmentionnée, ou un fragment ou une séquence dérivée de cette dernière, tels que définis ci-dessus, et, le cas échéant, contenant une ou plusieurs séquence(s) d'ADN codant pour tout ou partie d'une enzyme autre que la CCR, telle que définie ci-dessus.
- soit à l'aide de plusieurs vecteurs recombinants dont l'un au moins contient une séquence d'ADN selon l'invention susmentionnée, ou un fragment ou une séquence dérivée de cette dernière, tels que définis ci-dessus, tandis que l' (ou les) autre(s) vecteur(s) recombinant(s) contien(nen)t une séquence d'ADN codant pour tout ou partie d'une enzyme autre que la CCR, telle que définie ci-dessus.

D'une manière générale, toujours si l'on considère que la teneur moyenne normale en lignines d'une plante varie entre environ 15% et environ 35% en poids de matière sèche, l'augmentation de la teneur en lignines résultant de la mise en oeuvre du procédé susmentionné, est avantageusement telle que les plantes ainsi transformées présentent une teneur moyenne en lignines variant entre environ 20% et environ 40%, ou encore entre environ 18% et environ 38%.

L'invention vise plus particulièrement l'application du procédé susmentionné d'augmentation des teneurs en lignines dans les plantes (encore désigné procédé de surexpression du gène de la CCR), à l'obtention de plantes transformées génétiquement, présentant des teneurs en lignines augmentées par rapport aux teneurs normales en lignines chez ces plantes, et dont les propriétés de résistance à des attaques de l'environnement, notamment à des attaques parasitaires, se trouvent être ainsi améliorées par rapport à ces mêmes plantes non transformées. Il est particulièrement avantageux dans ce dernier cas, d'utiliser en association avec le gène CCR, ou une séquence dérivée, dans les vecteurs susmentionnés, des promoteurs spécifiques particulièrement exprimés dans les tissus de surface et/ou en réponse à la blessure.

Par ailleurs, l'invention concerne également l'application du procédé susmentionné de surexpression du gène de la CCR, à l'amélioration de la croissance des plantes ainsi transformées génétiquement, notamment dans certains domaines tels que l'horticulture ou l'arboriculture, où il est souhaitable d'obtenir des plantes de dimension réduite.

Enfin, les cycles benzéniques de la lignine ont une plus grande énergie intrinsèque que les chaînes aliphatiques des résidus glucose de la cellulose. Ainsi, l'augmentation de la proportion de lignines chez les végétaux utilisés comme combustibles, selon le procédé susmentionné de l'invention, permet d'améliorer le potentiel énergétique de ces végétaux combustibles ainsi transformés.

Dans les deux cas de régulation négative ou de surexpression de la CCR, il est tout à fait envisageable que la modulation de cette activité se répercute sur la teneur en lignines des plantes transformées. En effet, la CCR dont le niveau d'activité est très bas dans la plante, semble constituer l'enzyme régulatrice de la synthèse des lignines.

S'agissant des techniques de transformation utilisées pour la mise en oeuvre d'un des procédés décrits ci-dessus de l'invention, on aura avantageusement recours aux techniques suivantes:
A) La technologie de transformation par l'intermédiaire du plasmide Ti d'*Agrobacterium tumefaciens* décrite par Bevan (1984) Nucleic Acid Research, **12**: 8711-8721. Elle fait appel essentiellement à la méthode de co-culture, et fait intervenir une co-transformation avec un gène de sélection pour pouvoir repérer les transformants.
   Elle est particulièrement applicable aux dicotylédones, ex.: tabac, luzerne, colza.
B) La technique de transfert direct de gènes par biolistique décrite en détail par (Zumbrum et al., 1989, Technique **1**, 204-216; Sanford et al., 1991, Technique **3,** 3-16).

Cette technique implique l'association de l'ADN recombinant selon l'invention à des microparticules d'or ou de tungstène qui sont propulsées à l'aide d'un canon à particules sur le tissu à transformer. Elle sera particulièrement appliquée à la transformation d'espèces réfractaires aux agrobactéries.

Dans les deux cas susmentionnés, la vérification de la présence de l'ADN recombinant selon l'invention sera réalisée par des expériences d'hybridation de type southern et d'amplification génique (polymerase chain reaction), à l'aide de sondes et d'amorces oligonucléotidiques issues notamment de la séquence SEQ ID NO 1 ou SEQ ID NO 3.

L'invention concerne également les cellules de plantes transformées par un vecteur selon l'invention, notamment par les techniques décrites ci-dessus, et comprenant une séquence d'ADN selon l'invention intégrée de façon stable dans leur génome.

L'invention vise également les plantes transformées telles qu'obtenues par culture des cellules transformées susmentionnées.

Les plantes transformées peuvent être ensuite propagées par voie sexuelle ou par voie végétative *in vitro* ou *in natura*.

L'invention a également pour objet les fragments de plantes. notamment fruits, semences, pollen, transformés par incorporation dans leur génome d'une séquence d'ADN selon l'invention, à l'aide des vecteurs recombinants susmentionnés.

L'invention concerne également les anticorps dirigés contre les polypeptides recombinants de l'invention, et plus particulièrement ceux dirigés contre les CCR recombinantes susmentionnées.

De tels anticorps peuvent être obtenus par immunisation d'un animal avec ces polypeptides suivie de la récupération des anticorps formés.

Il va de soi que cette production n'est pas limitée aux anticorps polyclonaux.

Elle s'applique encore à tout anticorps monoclonal produit par tout hybridome susceptible d'être formé, par des méthodes classiques, à partir des cellules spléniques d'un animal, notamment de souris ou de rat, immunisés contre l'un des polypeptides purifiés de l'invention d'une part, et des cellules d'un myélome approprié d'autre part, et d'être sélectionné, par sa capacité à produire des anticorps monoclonaux reconnaissant le polypeptide susmentionné initialement mis en oeuvre pour l'immunisation des animaux.

L'invention vise également l'utilisation des anticorps susmentionnés dirigés contre les polypeptides recombinants de l'invention, pour la mise en oeuvre d'une méthode de détection ou de dosage des CCR chez les plantes, à partir d'échantillons prélevés chez ces dernières.

Il convient de préciser que sont exclues des séquences nucléotidiques de l'invention et de leur utilisation susmentionnée, les séquences nucléotidiques représentées par SEQ ID NO 5, SEQ ID NO 7, SEQ ID NO 9 et SEQ ID NO 11, codant respectivement pour la CCR d'eucalyptus représentée par SEQ ID NO 6, la CCR de peuplier représentée par SEQ ID NO 8, la CCR de fétuque représentée par SEQ ID NO 10, et la CCR de tabac représentée par SEQ ID NO 12, ainsi que la séquence représentée par SEQ ID NO 13 codant pour la protéine représentée par SEQ ID NO 14 dérivée de la CCR d'eucalyptus susmentionnée.

De même, sont exclues des séquences nucléotidiques de l'invention et de leur utilisation susmentionnée, les séquences complémentaires des séquences nucléotidiques SEQ ID NO5, SEQ ID NO 7, SEQ ID NO 9, SEQ ID NO 11 et SEQ ID NO 13, ainsi que les fragments ou séquences dérivées de ces séquences nucléotidiques ou de leurs séquences complémentaires, dans la mesure où ces fragments et séquences dérivées sont identiques aux fragments et séquences dérivées, tels que définis ci-dessus, des séquences nucléotidiques représentées par SEQ ID NO 1 et SEQ ID NO 3 ou de leurs séquences complémentaires.

Sont également exclus du cadre de la présente invention :
- les ARNm codés par les séquences d'ADN représentées par SEQ ID NO 5, SEQ ID NO 7, SEQ ID NO 9, SEQ ID NO 11 et SEQ ID NO 13, ou codés par un fragment ou une séquence dérivée de ces séquences d'ADN, dans la mesure où ce fragment ou séquence dérivée sont identiques aux fragments et séquences dérivées, tels que définis ci-dessus, des séquences représentées par SEQ ID NO 1 et SEQ ID NO 3,
- les ARNm antisens constitués de nucléotides complémentaires des ARNm susmentionnés,
- les polypeptides représentés par SEQ ID NO 6, SEQ ID NO 8, SEQ ID NO 10, SEQ ID NO 12 et SEQ ID NO 14, ainsi que tout fragment ou séquence dérivée des polypeptides susmentionnés, dans la mesure où ce fragment ou séquence dérivée sont identiques aux fragments et séquence dérivée, tels définis ci-dessus, des séquences polypeptidiques représentées par SEQ ID NO 2 et SEQ ID NO 4.

L'invention sera davantage détaillée dans la description qui suit de l'obtention de la CCR sous forme purifiée chez l'eucalyptus, et de l'ADNc codant pour la CCR d'eucalyptus, de luzerne et du maïs.

### A) Obtention de la CCR d'eucalyptus purifiée, et de l'ADNc codant pour une CCR d'eucalyptus.

### I Purification de la CCR d'eucalyptus.

La CCR a fait l'objet d'un nombre très restreint d'études. Parmi les quelques publications la concernant, on peut citer:
Wengenmayer H., Ebel J., Grisebach H., 1976 - Enzymatic synthesis of lignin precursors, purification and properties of a cinnamoyl CoA: NaDPH reductase from cell suspension cultures from soybean (*Glycine max*), Eur. J. Biochem., **65:** 529-536.
Luderitz T., Grisebach H., 1981 - Enzymatic synthesis of lignin precursors, comparison of cinnamoyl: CoA reductase and cinnamyl alcohol dehydrogenase: NADP dehydrogenase from spruce (*Picea abies* L.) and soybean (*Glycine max L.*), Eur. J. Biochem., **119**: 115-127.
Sarni F., Grand C., Boudet A.M., 1984 - Purification and properties of cinnamoyl: CoA reductase and cinnamyl alcohol dehydrogenase from poplar stems (*Populus x euramericana*). Eur. J. Biochem., **139**: 259-265.

Le travail décrit ci-après a contribué à définir un protocole de purification original, simple et rapide de la CCR d'eucalyptus. Ce protocole est également plus efficace que ceux décrits précédemment dans la littérature. En effet, il a permis pour la première fois, l'obtention de quantités d'enzyme purifiée à homogénéité, suffisantes pour obtenir des séquences peptidiques internes et conduire à terme au clonage de l'ADNc correspondant.

Toutes les étapes de purification de la CCR ont été réalisées à 4°C.

### 1. Obtention d'un extrait brut de xylème d'eucalyptus.

Le matériel végétal a été obtenu par "grattage" d'une fraction tissulaire enrichie en xylème de branches d'*Eucalyptus gunnii* âgés de 5 ans.

300 g de xylème préalablement congelé dans l'azote liquide ont été réduits en poudre à l'aide d'un moulin à café. Le broyat ainsi obtenu a été homogénéisé dans un litre de tampon d'extraction (100 mM Tris-HCl pH 7.6, 2% PEG 6000, 5 mM DTT, 2% PVPP), filtré sur deux épaisseurs de Miracloth, et amené à 30% de la saturation en sulfate d'ammonium. Après une centrifugation de 30 minutes à 15000xg, le culot obtenu est remis en suspension dans 60 ml de tampon 1 [20 mM Tris-HCl pH 7.5, 5 mM DTT (dithiothreitol), 5% éthylène glycol]. L'extrait ainsi obtenu est "clarifié" par une centrifugation de 15 min à 10 000xg, puis dessalé par passage sur une Sephadex G25 équilibrée dans le tampon 1.

### 2. Chromatographie d'affinité sur Red Sepharose.

L'extrait brut dessalé est déposé sur une colonne d'affinité "Red Sepharose" (1.5 x 19 cm, Pharmacia), équilibrée dans le tampon 1. Après un premier rinçage de la colonne par 50 ml de tampon 1, les protéines sont éluées par un gradient linéaire de Tris de 20 mM à 1.5 M Tris-HCl pH7.5, contenant 5 mM DTT, 5% éthylène glycol. Le volume total du gradient est de 200 ml et le débit de 36 ml/h. les fractions présentant une activité CCR sont regroupées et dessalées par passage sur une colonne de Séphadex G25, équilibrée dans le tampon 1.

### 3. Chromatographie d'échange d'anions sur MonoQ.

Les fractions ainsi regroupées et dessalées sont chromatographiées sur une colonne d'échange d'anions MonoQ (HR 5/5, Pharmacia). L'élution des protéines est effectuée par l'application d'un gradient linéaire de 20 à 300 mM en Tris-HCl pH 7.5, contenant 5% d'éthylène glycol et 5 mM DTT. Le volume total du gradient est de 50 ml et le débit de 1 ml/min. Comme à l'étape précédente, les fractions contenant l'enzyme CCR active sont regroupées et dessalées, mais dans ce cas le tampon d'équilibration des colonnes de Sephadex G25 est un tampon phosphate 20 mM pH 7.6, contenant 5 mM DTT (tampon 2).

### 4. Chromatographie d'affinité sur "Mimetic Red".

Le groupe de fractions CCR ainsi obtenu est déposé sur une colonne Mimetic Red 2 A6XL (ACL, Cambridge). La colonne est préalablement lavée avec 30 ml de tampon 2 contenant 8 mM NAD. Ce lavage a pour but d'éliminer des enzymes fonctionnant spécifiquement avec le NAD comme cofacteur, telle que la malate deshydrogénase qui copurifie avec la CCR dans les étapes précédentes. L'élution spécifique de la CCR est obtenue par application d'un gradient (15 ml) de NADP 0-8 mM dans le tampon 2. Les fractions contenant la CCR pure et active sont conservées à -80°C après addition d'un stabilisateur (éthylène glycol à la concentration finale de 5%).

L'enzyme purifiée ainsi obtenue présente une activité spécifique de 451 nKat/mg de protéine, en utilisant le feruloyl CoA comme substrat. Le rendement obtenu (36 µg de protéine pure pour 300 g de matériel végétal de départ) ne reflète pas la proportion de CCR *in planta*, en effet dans un souci majeur d'éliminer le maximum de contaminants à chaque étape de purification, seules les fractions présentant une très forte activité CCR sont traitées par l'étape suivante. Le facteur de purification obtenu par ce protocole est de 282.

### II Caractérisation de la CCR.

La CCR d'eucalyptus est un monomère de 38kD comme en témoignent les résultats convergents obtenus pour la taille de l'enzyme native par chromatographie d'exclusion sur Superose 6 (Pharmacia), et pour la taille de la sous-unité monomère sur gel d'électrophorèse dénaturant. Le point isoélectrique, estimé par chromatographie sur MonoP (Pharmacia) est proche de 7.

La recherche du pH et du tampon optimum fait ressortir que la mesure de l'activité CCR telle qu'elle a été initialement décrite (Luderitz et Grisebach, 1981), est parfaitement adaptée à la mesure de l'activité CCR d'eucalyptus (tampon phosphate 100 mM, pH 6.25).

La pureté de la CCR, présente à l'état d'une bande unique sur gel d'électrophorèse monodimensionnelle (SDS PAGE) a été confirmée par l'obtention d'une seule tache ("spot") après électrophorèse bidimensionnelle et coloration à l'argent.

### III Obtention de l'ADNc codant pour la CCR d'eucalyptus

Afin de s'affranchir d'un éventuel problème de contamination résiduelle non détectable, l'enzyme pure a été soumise à une électrophorèse préparative en conditions semi-dénaturantes et digérée *in situ* dans le gel. La digestion a été réalisée à l'aide d'endolysine C qui coupe spécifiquement les protéines après les résidus lysine, permettant l'obtention de peptides relativement longs. Les peptides résultant de la digestion ont été séparés en phase reverse sur HPLC et certains d'entre eux ont été séquences à l'aide d'un microséquenceur de protéines (Applied Biosystems 470). Les séquences de ces peptides internes figurent ci-dessous: X représentant tout acide aminé

L'ADNc codant pour la CCR a été obtenu par criblage à l'aide d'oligonucléotides d'une banque d'ADNc construite dans le phage λ ZAPII (vecteur commercialement disponible, Stratagène) à partir de messagers extraits de xylème d'*Eucalyptus gunnii*. 600 000 phages ont été criblés à l'aide d'un groupe d'oligonucléotides dégénérés marqués à l'extrémité 3' au phosphore 32, à l'aide d'une terminal transferase. Les séquences des oligonucléotides utilisés pour le criblage ont été déterminées à partir des séquences peptidiques internes susmentionnées. Ces peptides ayant été générés par coupure à l'endolysine C, une lysine a été rajoutée en première position pour permettre l'élaboration d'oligonucléotides à moindre dégénérescence. En effet, cet acide aminé qui ne peut être codé que par deux codons, fait partie des acides aminés dont le code est le moins dégénéré et par conséquent convient tout à fait à l'élaboration d'oligonucléotides à partir de séquences peptidiques.

Les séquences des oligonucléotides utilisés pour le criblage de la banque de cDNA d'eucalyptus dérivées des acides aminés soulignés (I = inosine), sont indiquées ci-après:

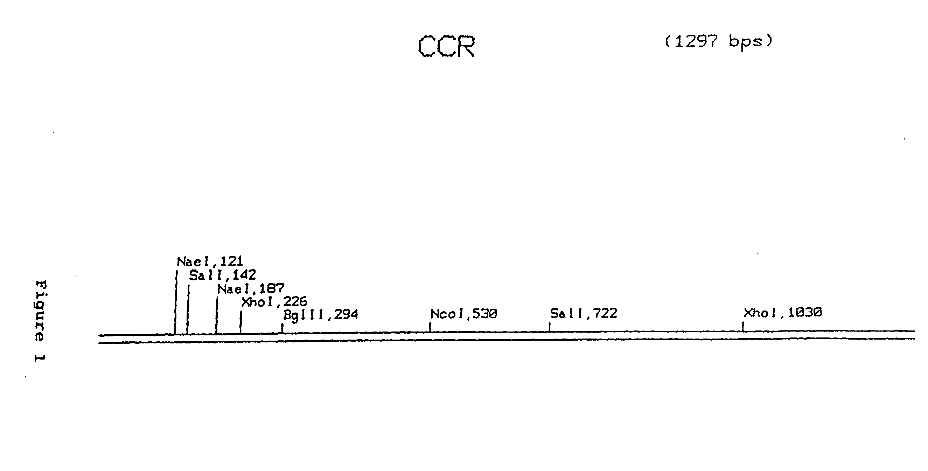

Les conditions d'hybridation utilisées pour le criblage sont les suivantes: la préhybridation est effectuée pendant 6 à 7 heures dans du 5XSSPE, 0.25 % poudre de lait écrémé, 0.05% SDS (sodium dodecyl sulfate) à 42°C. L'hybridation est réalisée dans cette même solution, en présence des 4 oligonucléotides marqués en 3' au ddATPα³²P, pendant 24 heures à 42°C. Au terme de ces 24 heures d'hybridation, les filtres sont lavés trois fois pendant 15 minutes dans du 2XSSC, 0.1% SDS puis mis en contact avec un film autoradiographique pendant 24 heures à -80°C. Les phages hybridant avec le groupe d'oligonucléotides ont été purifiés par 2 tours supplémentaires de criblage ("plaque purification"). Une fois purifiés, les six clones positifs ont été testés avec chacun des oligonucléotides pris indépendamment. Un phage a réagi positivement avec les 4 oligonucléotides, il a été traité de manière à "exciser" le plasmide Bluescript recombinant en suivant les indications du fabricant (Stratagène). La carte de restriction de l'insert (codant pour la CCR) contenu dans ce plasmide est schématisée sur la figure 1.

### IV Caractérisation et identification de l'ADNc de la CCR

La séquence en acides aminés (représentée par SEQ ID NO 6) déduite de la séquence nucléotidique (représentée par SEQ ID NO 5) code pour une protéine de 335 acides aminés dont le poids moléculaire est de 36,5 kD et le point isoélectrique d'environ 5,8. Il est important de souligner que toutes les séquences peptidiques obtenues à partir de la CCR purifiée sont retrouvées dans la séquence peptidique déduite de la séquence nucléotidique de l'ADNc.

Des recherches d'homologies avec des clones déjà existants ont été effectuées en utilisant les programmes BLAST et FASTA dans toutes les banques protéiques et nucléiques disponibles. Une homologie significative a été trouvée avec une autre réductase du métabolisme des composés phénoliques, la dihydroflavonol réductase (DFR). L'identité est d'environ 40% et la similarité proche de 20% entre la séquence peptidique déduite de l'ADNc de la CCR et les séquences des diverses dihydroflavonol réductase répertoriées dans les banques, ce qui confirme que le clone identifié est différent d'un clone codant pour une DFR.

### V Production de CCR recombinante active dans E. Coli.

Pour aller plus loin dans l'identification de l'ADNc de la CCR, la protéine recombinante a été produite dans *E*. *Coli* et son activité enzymatique a été recherchée. Les détails expérimentaux de cette approche sont décrits ci-après.

### 1- Introduction de l'ADNc dans le vecteur d'expression pT7-7.

Afin de pouvoir cloner l'ADNc dans le vecteur d'expression pT7-7 (disponible commercialement), sous le contrôle du promoteur de la T7 polymérase, nous avons du introduire un site Ndel à l'ATG de l'ADNc. Ceci a été réalisé à l'aide d'une Taq polymérase lors d'une réaction d'amplification génique par PCR (Polymerase Chain reaction) entre un oligonucléotide muté et une amorce commerciale, T7, situé sur Bluescript en aval de l'extrémité 3' de l'ADNc. Le produit d'amplification obtenu est digéré par Kpnl, ce site est ensuite réparé à l'aide du fragment klenow de l'ADN polymérase I avant de soumettre le fragment à une digestion par Ndel, puis le fragment obtenu comportant un site Ndel en 5' et une extrémité franche en 3' est insérée à l'aide d'une ADN T4 ligase dans le vecteur pT7-7 préalablement ouvert par Ndel et Smal.

La séquence de l'oligonucléotide muté susmentionné est indiquée ci-après.

Les bases soulignées et en italiques ont été modifiées par rapport à la séquence initiale permettant la création d'un site Ndel (CATATG):

### 2. Surexpression de CCR dans E. Coli BL21

La construction ainsi obtenue est introduite dans la souche *E. Coli* BL21 (disponible commercialement) qui porte sur son chromosome le gène de la T7 polymérase sous contrôle du promoteur lac UV5, promoteur inductible par l'IPTG. La culture recombinante est cultivée à 37°C jusqu'à obtention d'une DO mesurée de 1 à 600 nm, puis la production de la CCR est induite par l'ajout d'IPTG (0.25% final) dans le milieu de culture. Des prélèvements sont réalisés à différents temps après induction et les cellules sont lysées selon le protocole décrit par Grima-Pettenati et al. (1993). Après centrifugation le surnageant contenant les protéines solubles, est utilisé pour mesurer l'activité CCR et pour visualiser la production de CCR après électrophorèse en conditions dénaturantes. On note l'apparition d'un polypeptide d'environ 38 kD dont l'intensité croit avec le temps post-induction et qui n'existe pas dans les témoins négatifs (souche BL21 contenant seulement le vecteur pT7-7 sans insert). De plus, la preuve finale de l'identité du clone CCR est apporté par la mesure d'une activité CCR (environ 7 nKat/ml de culture après 3h d'induction à 37°C) dans les extraits protéiques provenant des souches BL21 contenant le pT7-7 + ADNc CCR, seulement.

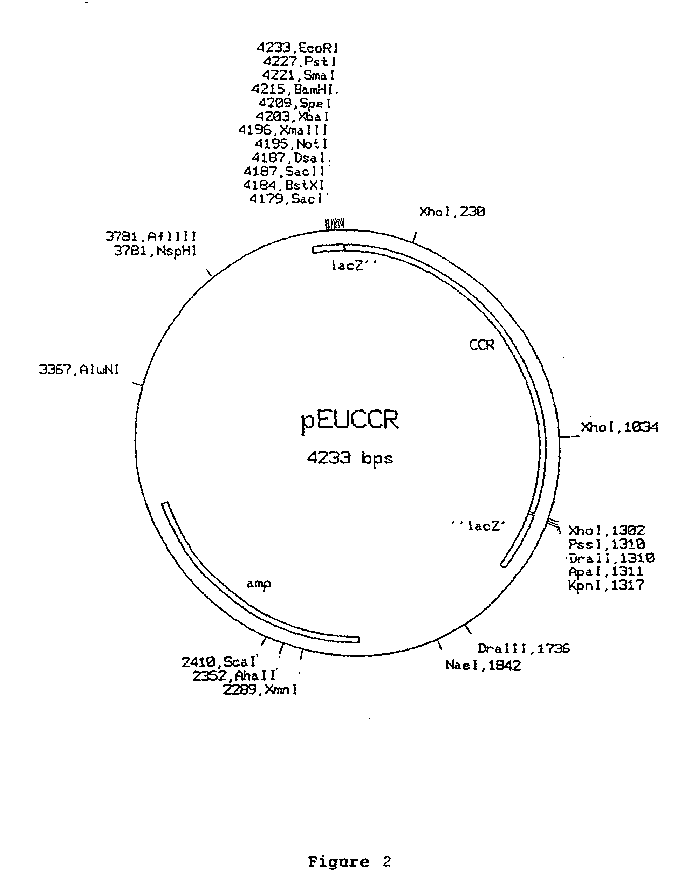

Le vecteur dénommé pEUCCR (représenté sur la figure 2), comprenant la séquence représentée par SEQ ID NO 5 clonée dans le vecteur Bluescript, a été déposé en culture dans des cellules de *E. coli* DH5α à la Collection Nationale de Culture de Micro-organismes (CNCM) de l'Institut Pasteur à Paris (France), le 17 mars 1994 sous le n° I-1405.

Légendes des figures:
Figure 1: carte de restriction de l'ADNc codant pour la CCR d'eucalyptus.
Figure 2: représentation schématique du plasmide pEUCCR contenant la séquence représentée par SEQ ID NO 5 (et identifiée par CCR dans le plasmide pEUCCR).
Figure 3: représentation schématique de la construction d'un vecteur contenant une séquence d'ADN codant pour la CCR d'eucalyptus selon l'invention (ou vecteur CCR sens).
Figure 4: représentation schématique de la construction d'un vecteur contenant une séquence d'ADN codant pour un ARN antisens susceptible de s'hybrider avec l'ARNm codant pour la CCR d'eucalyptus selon l'invention (ou vecteur CCR antisens).

### Concernant l'ADN "source servant à la construction d'un vecteur antisens (ou sens)

L'ARN antisens dérive préférentiellement de la séquence contenue dans le clone pEUCCR. Cette séquence peut être obtenue de différentes manières:
**1**) en coupant avec des enzymes de restriction appropriées la séquence d'ADN (ADNc) de la CCR contenue dans pEUCCR,
**2**) en réalisant une amplification génique (PCR) à l'aide d'oligonucléotides définis de manière à synthétiser le fragment d'ADN souhaité.

Le fragment d'ADN ainsi obtenu est cloné dans un vecteur d'expression des plantes en aval d'un promoteur et en amont d'un terminateur. Le clonage est réalisé de telle façon que le fragment d'ADN est inséré en orientation inverse par rapport au promoteur. Dans ce nouveau vecteur, le brin qui était initialement le brin matrice devient le brin codant et vice versa.

Le nouveau vecteur code pour un ARN dont la séquence est complémentaire de la séquence de l'ARN messager déduit de la séquence contenue dans pEUCCR.

Ainsi les 2 ARN sont complémentaires de par leur séquence mais aussi de par leur orientation (5'-3').

Comme source d'ADN pour la transcription de l'ARN antisens il est pratique d'utiliser un clone d'ADNc tel que celui contenu dans pEUCCR.

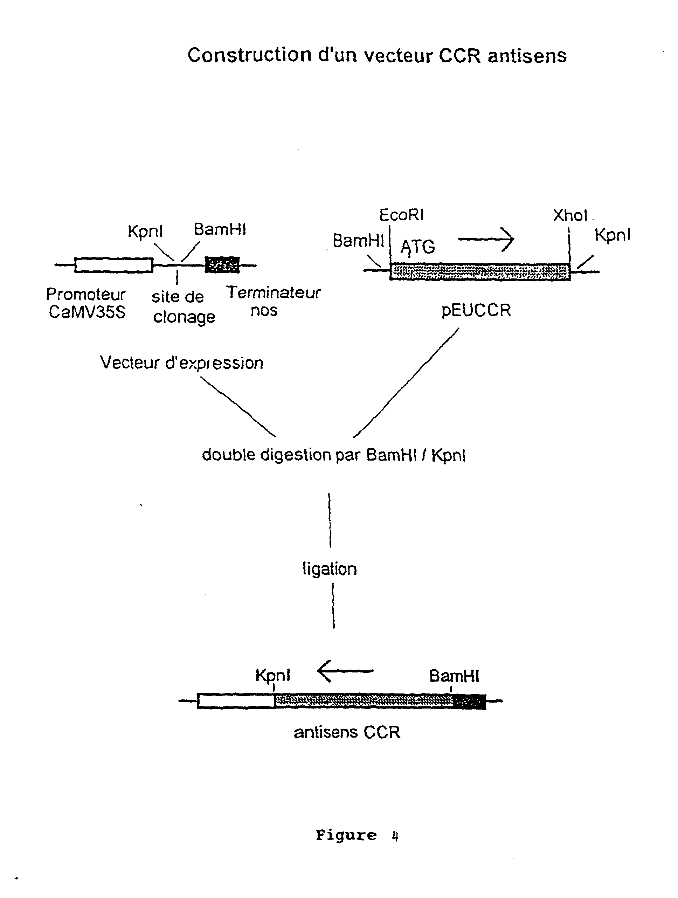

### Exemple de clonage antisens (cf. figure 4)

L'ADNc de la CCR est obtenu par une double digestion (BamHI et KpnI) à partir du vecteur pEUCCR. Le fragment d'ADN ainsi libéré est séparé physiquement du vecteur de clonage par une électrophorèse en gel d'Agarose (Bluescript).

La partie de gel renfermant ce fragment d'ADN est découpée et traitée de façon à obtenir l'ADN purifié (plusieurs méthodes peuvent être utilisées dont la "Low melting Agarose", décrite dans Sambrook et al. susmentionné, le Gene Clean, dont le kit est disponible commercialement).

Le fragment portant les extrémités BamHI et KpnI est "ligué" avec un vecteur d'expression de plantes préalablement digéré par ces mémes enzymes choisies de façon à ce que le cDNA soit inséré en orientation inverse par rapport au promoteur ³⁵S. Le brin qui sera transcrit dans les plantes sera dans ce cas le brin non codant.

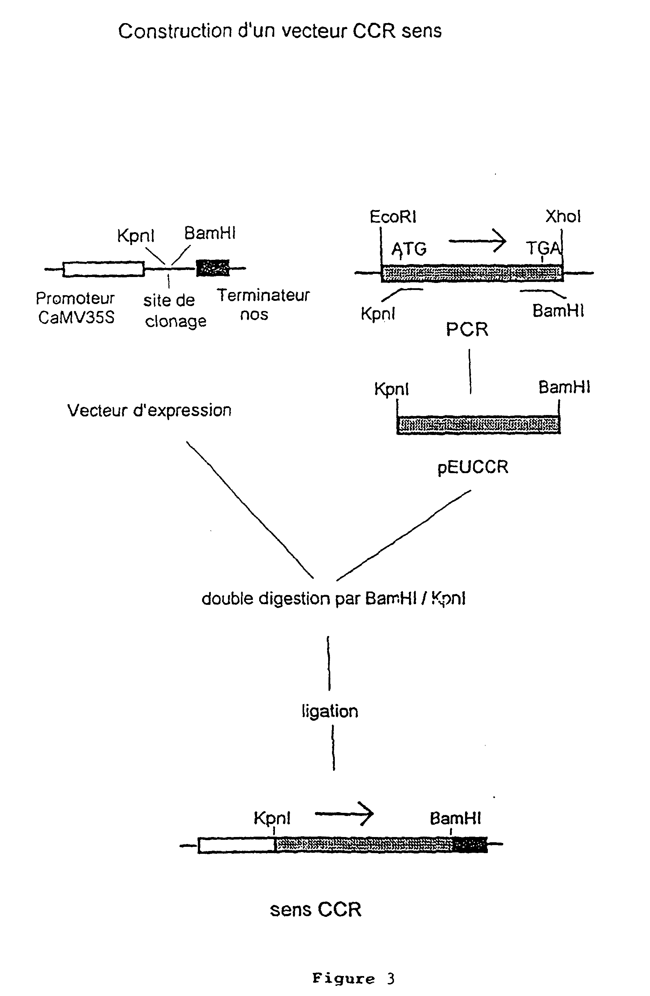

### Exemple de clonage sens (cf. figure 3)

Dans ce cas, il n'existe pas de sites de restriction "pratiques" pour réaliser une fusion traductionnelle avec le promoteur ³⁵S du vecteur d'expression. De nouveaux sites plus commodes ont été insérés à l'aide de la technique d'amplification génique (PCR). Deux oligonucléotides ont été définis en 5' et 3' de l'ADNc auxquels ont été ajoutées les séquences des sites reconnus par KpnI et BamHI (NB.: ce sont les mêmes sites qui ont été utilisés pour le clonage antisens susmentionné, mais positionnés différemment par rapport à l'orientation 5'-3').

L'amplification génique conduit à l'obtention d'un fragment contenant la totalité de la séquence codante du cDNA flanquée de 2 sites de restriction. La suite de la procédure est identique à celle décrite pour la construction antisens.

Mais, dans ce cas, on a réalisé une fusion du promoteur en phase avec l'ATG de la CCR qui doit conduire à une surexpression de l'ARN messager et donc de la protéine CCR.

Les exemples de clonage des séquences sens et antisens décrits ci-dessus dans le cas de la CCR d'eucalyptus, sont également applicables dans le cas de la CCR de luzerne et de celle de maïs.

### B) Obtention de l'ADNc codant pour la CCR de luzerne (Medicago truncatula).

### Caractéristiques de la banque d'ADNc :

La banque utilisée a été construite à partir d'ARN totaux extraits de racines de *Medicago truncatula*, dans le vecteur λZAPH (kit "ZAP-cDNA synthesis" de Stratagene).

### Criblage de la banque d'ADNc :

### Sonde :

Le criblage de la banque de luzerne a été effectué à l'aide de l'ADNc codant pour la CCR d'eucalyptus. Un fragment de 800 bp (Xho-Xho) de pEUCCR marqué par la technique d'amorçage aléatoire a servi de sonde.

Etalement de la banque et empreintes sur filtre de nitrocellulose :

300 000 clones ont été étalés puis transférés sur filtre de nitrocellulose (Schleicher & Schuell). Pour cela, les filtres ont été placés 5 mn sur les boîtes de culture puis immergés successivement dans les solutions suivantes :

| | |
|---|---|
| 1,5M NaCl/0, 5M NaOH | 5 mn |
| 1,5M NaCl/0, 5M Tris pH 8 | 5 mn |
| 3x SSC | 2 mn |
| cuisson 2 heures à 80°C. | |

### Préhybridation-hybridation :

Les filtres ont été préhybridés pendant 12 heures, puis hybridés pendant 24 heures à 37°C dans le milieu suivant :
Milieu de préhybridation et d'hybridation :
   Formamide 20%
   Dextran 10%
   NaCl 1M
   ADN sperme de saumon (1 mg/ml)
   0,2% polyvinyl-pyrralidone
   0,2% BSA
   0,2% ficoll
   0,05M Tris-HCl pH 7.5
   0,1% sodium pyrophosphate
   1% SDS.

Après hybridation, les filtres ont été lavés 2x 10 mn à température ambiante dans du 2x SSC-1% SDS, puis 2x 30 mn à 55°C dans la même solution.

Après exposition autoradiographique des filtres, 15 plages de lyse positives ont été identifiées. Ces plages de lyse ont été purifiées par 2 cycles supplémentaires de criblage, dans les conditions d'hybridation décrites ci-dessus.

### Excision in vivo :

A partir des clones positifs, le plasmide Bluescript du phage λ a été excisé selon le protocole d'excision *in vivo* du "ZAP-cDNA Synthesis kit".

### L'ADNc CCR de luzerne :

L'ADNc codant pour la CCR de luzerne, d'une taille de 1404 pbs, est inséré entre les sites EcoR1 (côté 5') et Xho (côté 3') du vecteur Bluescript. Il est constitué des parties suivantes :
- une partie 5' transcrite non traduite de 167 pbs,
- une région de 1028 pbs qui code pour une protéine de 342 acides aminés,
- une partie 3' transcrite non traduite de 209 pbs.

L'ADNc obtenu est représenté par SEQ ID NO 1, et la séquence en acides aminés déduite de cet ADNE est représenté par SEQ ID NO 2.

### C) Obtention de l'ADNc codant pour la CCR de maïs.

### Caractéristiques de la banque d'ADNc :

La banque utilisée a été construite à partir d'ARN totaux extraits de racines de maïs (variété AMO 406) carencé en fer, dans le vecteur λZAP (kit "ZAP-cDNA synthesis" de Stratagene).

### Criblage de la banque d'ADNc :

### Sonde :

Le criblage de la banque de maïs a été effectué à l'aide de l'ADNc CCR d'eucalyptus. Un fragment de 800 bp (Xho-Xho) de pEUCCR marqué par la technique d'amorçage aléatoire a servi de sonde.

Etalement de la banque et empreintes sur filtre de nitrocellulose :

500 000 clones ont été étalés puis transférés sur filtre de nitrocellulose (Schleicher & Schuell). Pour cela, les filtres ont été placés 5 mn sur les boîtes de culture puis immergés successivement dans les solutions suivantes :

| | |
|---|---|
| 1,5M NaCl/0, 5M NaOH | 5 mn |
| 1,5M NaCl/0, 5M Tris pH 8 | 5 mn |
| 3x SSC | 2 mn |
| cuisson 2 heures à 80°C. | |

### Préhybridation-hybridation :

Les filtres ont été préhybridés pendant 12 heures, puis hybridés pendant 24 heures à 55°C dans le milieu suivant :
Milieu de préhybridation et d'hybridation :
   3x SSC
   0,5% SDS
   0,1% lait en poudre
   ADN sperme de saumon (1 mg/ml).

Après hybridation, les filtres ont été lavés 2x 10 mn à température ambiante dans du 3x SSC-0,5% SDS, puis 2x 45 mn à 60°C dans la même solution.

Après exposition autoradiographique des filtres, 20 plages de lyse positives ont été identifiées. Ces plages de lyse ont été purifiées par 3 cycles supplémentaires de criblage, dans les conditions d'hybridation décrites ci-dessus.

### Excision in vivo :

A partir des clones positifs, le plasmide Bluescript du phage λ a été excisé selon le protocole d'excision *in vivo* du "ZAP-cDNA Synthesis kit".

L'ADNc obtenu est représenté par SEQ ID NO 3. et la séquence en acides aminés déduite de cet ADNc est représenté par SEQ ID NO 4.

## Revendications

1. Utilisation de séquences nucléotidiques recombinantes contenant une (ou plusicurs) région(s) codante(s), cette (ces) région(s) codante(s) étant constituée(s) d'une séquence nucléotidique choisie parmi les suivantes:
- la séquence nucléotidique représentée par SEQ ID NO 1, codant pour un ARNm, cet ARNm codant lui-même pour la cinnamoyl CoA réductase (CCR) de luzerne représentée par SEQ ID NO 2,
- la séquence nucléotidique représentée par SEQ ID NO 3, codant pour un ARNm, cet ARNm codant lui-même pour la CCR de maïs représentée par SEQ ID NO 4,
- la séquence nucléotidique complémentaire de celle représentée par SEQ ID NO 1 ou SEQ ID NO 3,
pour la transformation de cellules végétales en vue de l'obtention de plantes transgéniques au sein desquelles la biosynthèse des lignines est régulée soit dans le sens d'une augmentation, soit dans le sens d'une diminution des teneurs en lignines produites, par rapport aux teneurs normales en lignines produites chez les plantes.

2. Séquence d'ADN, **caractérisée en ce qu'**elle comprend à titre de région codante la séquence nucléotidique représentée par SEQ ID NO 1, codant pour un ARNm, cet ARNm codant lui-même pour la CCR représentée par SEQ ID NO 2.

3. Séquence d'ADN, **caractérisée en ce qu'**elle comprend à titre de région codante la séquence nucléotidique représentée par SEQ ID NO 3, codant pour ARNm, cet ARNm codant lui-même pour la CCR représentée par SEQ ID NO 4.

4. Séquence d'ADN, **caractérisée en ce qu'**elle comprend à titre de région codante la séquence nucléotidique complémentaire de celle représentée par SEQ ID NO 1.

5. Séquence d'ADN, **caractérisée en ce qu'**elle comprend à titre de région codante la séquence nucléotidique complémentaire de celle représentée par SEQ ID NO 3.

6. ARNm codé par une séquence d'ADN selon l'une des revendications 2 à 5, choisi parmi :
- l'ARNm codé par la séquence d'ADN représentée par SEQ ID NO 1, ledit ARNm étant susceptible de coder à son tour pour la CCR présente chez la luzerne, telle que représentée par SEQ ID NO 2,
- l'ARNm codé par la séquence d'ADN représentée par SEQ ID NO 3, ledit ARNm étant susceptible de coder à son tour pour la CCR présente chez le maïs, telle que représentée par SEQ ID NO 4.

7. ARNm antisens ayant pour effet de moduler la biosynthèse des lignines chez les plantes, **caractérisé en ce qu'**il comprend des nucléotides complémentaires des nucléotides constituant un ARNm selon la revendication 6.

8. CCR recombinante de luzerne ou de maïs, représentée par SEQ ID NO 2 ou SEQ ID NO 4 respectivement.

9. Séquences nucléotidiques codant pour les CCR représentées par SEQ ID NO 2 ou SEQ ID NO 4, lesdites séquences nucléotidiques étant **caractérisées en ce qu'**elles correspondent aux séquences représentées par SEQ ID NO 1 ou SEQ ID NO 3 respectivement, ou à toute séquence dérivée de ces demières par dégénérescence du code génétique, et étant néanmoins susceptibles de coder pour une CCR susmentionnée.

10. Complexes formés entre un ARNm antisens selon la revendication 7, et un ARNm selon la revendication 6.

11. Séquence nucléotidique recombinante, **caractérisée en ce qu'**elle comprend au moins une séquence d'ADN selon l'une des revendications 2 et 3, susceptible de coder pour un ARNm lui-même susceptible de coder pour une CCR chez la luzerne ou le maïs, ladite séquence selon l'une des revendications 2 et 3 étant insérée dans une séquence hétérologue.

12. Séquence nucléotidique recombinante, **caractérisée en ce qu'**elle comprend au moins une séquence d'ADN complémentaire selon l'une des revendications 4 et 5, insérée dans une séquence hétérologue, ladite séquence d'ADN complémentaire codant pour un ARNm antisens selon la revendication 7.

13. Séquence nucléotidique recombinante selon la revendication 11 ou la revendication 12, **caractérise en ce qu'**elle comprend les éléments nécessaires pour réguler l'expression de la séquence nucléotidique selon l'une des revendications 2 et 3, ou de sa séquence complémentaire selon l'une des revendications 4 et 5, notamment un promoteur et un terminateur de la transcription de ces séquences, et le cas échéant, au moins une séquence d'ADN codant pour une autre enzyme que la CCR, qui se trouve être impliquée dans une étape de biosynthèse des lignines chez les plantes, notamment l'ARNm codant lui-même pour l'alcool cinnamylique deshydrogénase (CAD), ou au moins une séquence codant pour l'ARNm antisens susceptible de s'hybrider avec l'ARNm susmentionné, notamment avec l'ARNm codant pour la CAD.

14. Vecteur recombinant **caractérisé en ce qu'**il comprend une séquence nucléotidique recombinante selon l'une des revendications 11 à 13, intégrée dans l'un de ses sites de son génome non essentiels pour sa réplication.

15. Procédé de régulation de la biosynthèse de lignines chez les plantes, soit par diminution, soit par augmentation des teneurs en lignines produites, par rapport aux teneurs normales en lignines produites chez des plantes, ledit procédé comprenant une étape de transformation de cellules de ces plantes à l'aide d'un vecteur selon la revendication 14.

16. Procédé de diminution de la biosynthèse de lignine chez les plantes, et donc de diminution des teneurs en lignines produites par rapport aux teneurs normales en lignines produites chez les plantes, **caractérisé en ce qu'**il est effectué par transformation du génome de ces plantes, en y incorporant:
- au moins une séquence d'ADN selon l'une des revendications 4 et 5,
- et, le cas échéant, au moins une séquence d'ADN codant pour un ARNm anlisens capable de s'hybrider à un ARNm codant pour une autre enzyme que la CCR, qui se trouve être impliquée dans une étape de la biosynthèse des lignines chez les plantes, notamment l'ARNm codant pour la CAD,
ladite transformation étant réalisée:
- soit à l'aide d'un vecteur recombinant selon la revendication 14, contenant une séquence nucléotidique recombinante sclon la revendication 12 ou la revendication 13,
- soit à l'aide de plusieurs vecteurs recombinants dont l'un au moins contient une séquence nucléotidique recombinante selon la revendication 12, tandis que l'(ou les) autre(s) vecteur(s) recombinant(s) contien(nen)t une séquence d'ADN codant pour un ARNm antisens capable de s'hybrider à un ARNm codant pour une autre enzyme que la CCR, telle que définie ci-dessus.

17. Procédé de diminution de la biosynthèse de lignine chez les plantes, et donc de diminution des teneurs en lignines produites par rapport aux teneurs normales en lignines produites chez les plantes, **caractérisé en ce qu'**il est effectué par transformation du génome de ces plantes, en y incorporant:
- au moins une séquence d'ADN selon la revendication 2 et 3,
- et, le cas échéant, au moins une séquence d'ADN codant pour une autre enzyme que la CCR, qui se trouve être impliquée dans une étape de la biosynthèse des lignines chez les plantes, notamment une séquence d'ADN codant pour la CAD,
ladite transformation étant réalisée:
- soit à l'aide d'un vecteur recombinant selon la revendication 14, contenant la séquence nucléotidique recombinante selon la revendication 11 ou la revendication 13,
- soit à l'aide de plusieurs vecteurs recombinants dont l'un au moins contient une séquence nucléotidique recombinante selon la revendication 11, tandis que l' (ou les) autre(s) vecteur(s) recombinant(s) contien(nen)t une séquence d'ADN codant pour une enzyme autre que la CCR, telle que définie ci-dessus.

18. Procédé d'augmentation de la biosynthèse de lignine chez les plantes, et donc d'augmentation des teneurs en lignines produites par rapport aux teneurs normales en lignines produites chez les plantes, **caractérisé en ce qu'**il est effectué par transformation du génome de ces plantes, en y incorporant:
- au moins une séquence d'ADN selon l'une des revendications 2 et 3,
- et, le cas échéant, au moins une séquence d'ADN codant pour une autre enzyme que la CCR, qui se trouve être impliquée dans une étape de la biosynthèse des lignines chez les plantes, notamment une séquence d'ADN codant pour la CAD,
ladite transformation étant réalisée:
- soit à l'aide d'un vecteur recombinant selon la revendication 14, contenant la séquence nucléotidique recombinante selon la revendication 11 ou la revendication 13,
- soit à l'aide de plusieurs vecteurs recombinants dont l'un au moins contient une séquence nucléotidique recombinante selon la revendication 11, tandis que l' (ou les) autre(s) vecteur(s) recombinant(s) contien(nen)t une séquence d'ADN codant pour une enzyme autre que la CCR, telle que définie ci-dessus.

19. Plantes ou fragments de plantes, notamment cellules, fruits, semences, pallen, transformés par incorpomtion dans leur génome d'au moins unc séquence nucléotidique selon l'une des revendications 2 à 5.

20. CCR recombinantes représentées par SEQ ID NO 2 ou SEQ ID NO 4, telles qu'obtenues par transformation de cellules végétales en intégrant de façon stable dans leur génome, une séquence nucléotidiqae recombinante selon l'une des revendications 11 à 13, notamment à l'aide d'un vecteur selon la revendication 14.

## Claims

1. Use of recombinant nucleotide sequences containing one (or more) coding region(s), this (these) coding region(s) being made up of a nucleotide sequence chosen from the following:
- the nucleotide sequence represented by SEQ ID NO 1 which codes for an mRNA, this mRNA itself coding for the cinnamoyl-CoA reductase (CCR) of lucerne represented by SEQ ID NO 2,
- the nucleotide sequence represented by SEQ ID NO 3 which codes for an mRNA, this mRNA itself coding for the CCR of maize represented by SEQ ID NO 4,
- the nucleotide sequence complementary to that represented by SEQ ID NO 1 or SEQ ID NO 3,
for the transformation of plant cells in order to obtain transgenic plants within which the biosynthesis of lignins is regulated either in the sense of an increase or in the sense of a reduction in the contents of lignins produced, with respect to the normal contents of lignins produced in the plants.

2. DNA sequence, **characterized in that** it contains, as the coding region the nucleotide sequence represented by SEQ ID NO 1 which codes for an mRNA, this mRNA itself coding for the CCR represented by SEQ ID NO 2.

3. DNA sequence, **characterized in that** it contains, as the coding region the nucleotide sequence represented by SEQ ID NO 3 which codes for mRNA, this mRNA itself coding for the CCR represented by SEQ ID NO 4.

4. DNA sequence, **characterized in that** it contains, as the coding region the nucleotide sequence complementary to that represented by SEQ ID NO 1.

5. DNA sequence, **characterized in that** it contains, as the coding region the nucleotide sequence complementary to that represented by SEQ ID NO 3.

6. mRNA coded by a DNA sequence according to one of claims 2 to 5, chosen among :
- the mRNA coded by the DNA sequence represented by SEQ ID NO 1, the said mRNA being capable of coding in its turn for the CCR present in lucerne, such as is represented by SEQ ID NO 2,
- the mRNA coded by the DNA sequence represented by SEQ ID NO 3, the said mRNA being capable of coding in its turn for the CCR present in maize, such as is represented by SEQ ID NO 4.

7. Antisense mRNA having the property of modulating the biosynthesis of lignins in plants, **characterized in that** it comprises nucleotides complementary to the nucleotides which make up an mRNA according to claim 6.

8. Recombinant CCR of lucerne or maize, represented by SEQ ID NO 2 or SEQ ID NO 4 respectively.

9. Nucleotide sequences which code for the CCRs represented by SEQ ID NO 2 or SEQ ID NO 4, the said nucleotide sequences being **characterized in that** they correspond to the sequences represented by SEQ ID NO 1 or SEQ ID NO 3 respectively, or to any sequence derived from the latter by degeneration of the genetic code, and being capable however of coding for a CCR mentioned above.

10. Complexes formed between an antisense mRNA according to claim 7 and an mRNA according to claim 6.

11. Recombinant nucleotide sequence, **characterized in that** it contains at least one DNA sequence according to one of claims 2 and 3 which is capable of coding for an mRNA which is itself capable of coding for a CCR in plants, the said sequence according to one of claims 2 and 3 being inserted into a heterologous sequence.

12. Recombinant nucleotide sequence, **characterized in that** it contains at least one complementary DNA sequence according to one of claims 4 and 5, inserted into a heterologous sequence, the said complementary DNA sequence coding for an antisense mRNA according to claim 7.

13. Recombinant nucleotide sequence according to claim 11 or claim 12, **characterized in that** it contains the elements necessary for regulation of the expression of the nucleotide sequence according to one of claims 2 and 3, or its complementary sequence according to one of claims 4 and 5, in particular a promoter and a terminator of the transcription of these sequences, and, where appropriate, at least one DNA sequence which codes for all or part of an enzyme other than the CCR, which is found to be involved in a stage of biosynthesis of lignins in plants, in particular the mRNA which itself codes for cinnamyl alcohol dehydrogenase (CAD), or at least one sequence which codes for all or part of the antisense mRNA which is capable of hybridizing with the abovementioned mRNA, in particular with the mRNA which codes for CAD.

14. Recombinant vector, **characterized in that** it comprises a recombinant nucleotide sequence according to one of claims 11 to 13 integrated in to one of its sites of its genome which are not essential for its replication.

15. Process for regulating the biosynthesis of lignins in plants, either by reducing or by increasing the contents of lignins produced, with respect to the normal contents of lignins produced in the plants, the said process comprising a stage of transformation of cells of these plants with the aid of a vector according to claim 14.

16. Process for reducing the biosynthesis of lignin in plants, and therefore reducing the contents of lignins produced with respect to the normal contents of lignins produced in the plants, **characterized in that** it is carried out by transformation of the genome of these plants, incorporating:
- at least one DNA sequence according to one of claims 4 and 5,
- and, where appropriate, at least one DNA sequence which codes for all or part of an antisense mRNA which is capable of hybridizing with an mRNA which codes for an enzyme other than the CCR, which is found to be involved in a stage of the biosynthesis of lignins in the plants, in particular the mRNA which codes for CAD,
the said transformation being realized:
- either with the aid of a recombinant vector according to claim 14 containing a recombinant nucleotide sequence according to claim 12 or claim 13,
- or with the aid of several recombinant vectors, at least one of which contains a recombinant nucleotide sequence according to claim 12, while the other recombinant vector(s) contain(s) a D NA sequence which c odes for an antisense mRNA which is capable of hybridizing with an mRNA which codes for an enzyme other than the CCR, as is defined above.

17. Process for reducing the biosynthesis of lignins in plants, and therefore reducing the contents of lignins produced with respect to the normal contents of lignins produced in the plants, **characterized in that** it is carried out by transformation of the genome of these plants, incorporating:
- at least one DNA sequence according to claim 2 and 3,
- and, where appropriate, at least one DNA sequence which codes for all or part of an enzyme other than the CCR, which is found to be involved in a stage of the biosynthesis of lignins in the plants, in particular a DNA sequence which codes for all or part of CAD,
the said transformation being carried out:
- either with the aid of a recombinant vector according to claim 14, containing the recombinant nucleotide sequence according to claim 11 or claim 13,
- or with the aid of several recombinant vectors, at least one of which contains a recombinant nucleotide sequence according to claim 11, while the other recombinant vector(s) contain(s) a DNA sequence which codes for all or part of an enzyme other than the CCR, as is defined above.

18. Process for increasing the biosynthesis of lignin in plants and therefore increasing the contents of lignins produced with respect to the normal contents of lignins produced in the plants, **characterized in that** it is carried out by transformation of the genome of these plants, incorporating:
- at least one DNA sequence according to one of claims 2 and 3,
- and, where appropriate, at least one DNA sequence which codes for an enzyme other than the CCR, which is found to be involved in a stage of the biosynthesis of lignins in the plants, in particular a DNA sequence which codes for CAD,
the said transformation being carried out:
- either with the aid of a recombinant vector according to claim 14, containing the recombinant nucleotide sequence according to claim 11 or claim 13,
- or with the aid of several recombinant vectors, at least one of which contains a recombinant nucleotide sequence according to claim 11, while the other recombinant vector(s) contain(s) a DNA sequence which codes for all or part of an enzyme other than the CCR, as is defined above.

19. Plants or fragments of plants, in particular cells, fruits, seeds or pollen, transformed by incorporation into their genome of at least one nucleotide sequence according to one of claims 2 to 5.

20. Recombinant CCRs represented by SEQ ID NO 2 or SEQ ID NO 4, such as are obtained by transformation of plant cells by integrating into their genome, in a stable manner, a recombinant nucleotide sequence according to one of claims 11 to 13, in particular with the aid of a vector according to claim 14.

## Patentansprüche

1. Verwendung von rekombinanten Nucleotidsequenzen, die eine (oder mehrere) codierende Region(en) enthalten, wobei diese codierende(n) Region(en) aus einer Nucleotidsequenz gebildet wird (werden), die aus den folgenden ausgewählt ist:
- die Nucleotidsequenz, die durch SEQ ID NO 1 dargestellt ist, die für eine mRNA codiert, die selbst für die Cinnamoyl-CoA-Reduktase (CCR) der Luzerne, dargestellt durch SEQ ID NO 2, codiert,
- die Nucleotidsequenz, die durch SEQ ID NO 3 dargestellt ist, die für eine mRNA codiert, die selbst für die CCR von Mais, dargestellt durch SEQ ID NO 4, codiert.
- die Nucleotidsequenz, welche zu jener, die durch SEQ ID NO 1 oder SEQ ID NO 3 dargestellt ist, komplementär ist,
für die Transformation von Pflanzenzellen, um transgene Pflanzen zu erhalten, in denen die Ligninbiosynthese im Sinne einer Erhöhung oder im Sinne einer Verringerung der Gehalte an produziertem Lignin im Vergleich zu den normalen Gehalten an in Pflanzen produziertem Lignin reguliert wird.

2. DNA-Sequenz, **dadurch gekennzeichnet, dass** sie als codierende Region die Nucleotidsequenz, die durch SEQ ID NO 1 dargestellt ist, enthält, welche für eine mRNA codiert, die selbst für die CCR codiert, die durch SEQ ID NO 2 dargestellt ist.

3. DNA-Sequenz, **dadurch gekennzeichnet, dass** sie als codierende Region die Nucleotidsequenz, die durch SEQ ID NO 3 dargestellt ist, enthält, welche für eine mRNA codiert, die selbst für die CCR codiert, die durch SEQ ID NO 4 dargestellt ist.

4. DNA-Sequenz, **dadurch gekennzeichnet, dass** sie als codierende Region die Nucleotidsequenz enthält, die komplementär zu jener ist, die durch SEQ ID NO 1 dargestellt ist.

5. DNA-Sequenz, **dadurch gekennzeichnet, dass** sie als codierende Region die Nucleotidsequenz enthält, die komplementär zu jener ist, die durch SEQ ID NO 3 dargestellt ist.

6. mRNA, die durch eine DNA-Sequenz gemäß einem der Ansprüche 2 bis 5 codiert ist, ausgewählt aus:
- der mRNA, die durch die DNA-Sequenz, die durch SEQ ID NO 1 dargestellt ist, codiert ist, wobei die mRNA ihrerseits für die CCR der Luzerne codieren kann, wie sie durch SEQ ID NO 2 dargestellt ist,
- der mRNA, die durch die DNA-Sequenz, die durch SEQ ID NO 3 dargestellt ist, codiert ist, wobei die mRNA ihrerseits für die CCR des Mais codieren kann, wie sie durch SEQ ID NO 4 dargestellt ist.

7. Antisense-mRNA, die eine Modulation der Biosynthese von Lignin bei Pflanzen bewirkt, **dadurch gekennzeichnet, dass** sie Nucleotide enthält, die komplementär zu Nucleotiden sind, die eine mRNA gemäß Anspruch 6 bilden.

8. Rekombinante CCR von Luzerne oder von Mais, die durch SEQ ID NO 2 bzw. SEQ ID NO 4 dargestellt ist.

9. Nucleotidsequenzen, die für die durch SEQ ID NO 2 oder SEQ ID NO 4 dargestellte CCR codieren, wobei die Nucleotidsequenzen **dadurch gekennzeichnet sind, dass** sie den Sequenzen, die durch SEQ ID NO 1 bzw. SEQ ID NO 3 dargestellt sind, oder Sequenzen, die von letzteren durch Degeneration des genetischen Codes abgeleitet wurden und dennoch für eine oben genannte CCR codieren können, entsprechen.

10. Komplexe, die zwischen einer Antisense-mRNA gemäß Anspruch 7 und einer mRNA gemäß Anspruch 6 gebildet sind.

11. Rekombinante Nucleotidsequenz, **dadurch gekennzeichnet, dass** sie mindestens eine DNA-Sequenz gemäß einem der Ansprüche 2 und 3 enthält, die für eine mRNA codieren kann, die selbst für eine CCR von Luzerne oder von Mais codieren kann, wobei die Sequenz gemäß einem der Ansprüche 2 und 3 in eine heterologe Sequenz inseriert ist.

12. Rekombinante Nucleotidsequenz, **dadurch gekennzeichnet, dass** sie mindestens eine komplementäre DNA-Sequenz gemäß einem der Ansprüche 4 und 5 enthält, die in eine heterologe Sequenz inseriert ist, wobei die komplementäre DNA-Sequenz für eine Antisense-mRNA gemäß Anspruch 7 codiert.

13. Rekombinante Nucleotidsequenz gemäß Anspruch 11 oder Anspruch 12, **dadurch gekennzeichnet, dass** sie die notwendigen Elemente zur Regulierung der Expression der Nucleotidsequenz gemäß einem der Ansprüche 2 und 3 oder ihrer komplementären Sequenz nach einem der Ansprüche 4 und 5, insbesondere einen Promotor und einen Terminator für die Transkription dieser Sequenzen, und gegebenenfalls mindestens eine DNA-Sequenz, die für ein anderes Enzym als CCR codiert, das mit einem Ligninbiosyntheseschritt bei Pflanzen in Zusammenhang steht, insbesondere mRNA, die selbst für Cinnamylalkoholdehydrogenase (CAD) codiert, oder mindestens eine Sequenz, die für eine Antisense-mRNA codiert, die fähig ist, mit der oben genannten mRNA, insbesondere mit der mRNA, die für die CAD codiert, zu hybridisieren, enthält.

14. Rekombinanter Vektor, **dadurch gekennzeichnet, dass** er eine rekombinante Nucleotidsequenz gemäß einem der Ansprüche 11 bis 13 enthält, die an einer seiner Stellen seines Genoms inseriert ist, die für seine Replikation nicht essentiell ist.

15. Verfahren zur Regulierung der Biosynthese von Lignin bei Pflanzen durch Verringerung oder Erhöhung der Gehalte an produziertem Lignin im Vergleich zu den normalen Gehalten an produziertem Lignin bei Pflanzen, wobei das Verfahren einen Schritt der Transformation der Zellen der Pflanzen mit Hilfe eines Vektors gemäß Anspruch 14 umfasst.

16. Verfahren zur Verringerung der Biosynthese von Lignin bei Pflanzen und somit zur Verringerung der Gehalte an produziertem Lignin im Vergleich zu den normalen Gehalten an produziertem Lignin bei Pflanzen, **dadurch gekennzeichnet, dass** dies durch Transformation des Genoms dieser Pflanzen bewirkt wird, indem dort eingebracht wird:
- mindestens eine DNA-Sequenz gemäß einem der Ansprüche 4 und 5,
- und gegebenenfalls mindestens eine DNA-Sequenz, die für eine Antisense-mRNA codiert, die fähig ist, mit einer mRNA, die für ein anderes Enzym als CCR codiert, das mit einem Ligninbiosyntheseschritt bei Pflanzen in Zusammenhang steht, insbesondere der mRNA, die für die CAD codiert, zu hybridisieren,
wobei die Transformation durchgeführt wird mit:
- einem rekombinanten Vektor gemäß Anspruch 14, der eine rekombinante Nucleotidsequenz gemäß Anspruch 12 oder Anspruch 13 enthält,
- oder mehreren rekombinanten Vektoren, von denen mindestens einer eine rekombinante Nucleotidsequenz gemäß Anspruch 12 enthält, wohingegen der (oder die) andere(n) rekombinante(n) Vektor(en) eine DNA-Sequenz enthält (enthalten), die für eine Antisense-mRNA codiert, die fähig ist, mit einer mRNA zu hybridisieren, die für ein anderes Enzym als CCR, wie oben definiert, codiert.

17. Verfahren zur Verringerung der Biosynthese von Lignin bei Pflanzen und somit zur Verringerung der Gehalte an produziertem Lignin im Vergleich zu den normalen Gehalten an produziertem Lignin bei Pflanzen, **dadurch gekennzeichnet, dass** dies durch Transformation des Genoms dieser Pflanzen bewirkt wird, indem dort eingebracht wird:
- mindestens eine DNA-Sequenz gemäß Anspruch 2 und 3,
- und gegebenenfalls mindestens eine DNA-Sequenz, die für ein anderes Enzym als CCR codiert, das mit einem Ligninbiosyntheseschritt bei Pflanzen in Zusammenhang steht, insbesondere eine DNA-Sequenz, die für die CAD codiert,
wobei die Transformation durchgeführt wird mit:
- einem rekombinanten Vektor gemäß Anspruch 14, der eine rekombinante Nucleotidsequenz gemäß Anspruch 11 oder Anspruch 13 enthält,
- oder mehreren rekombinanten Vektoren, von denen mindestens einer eine rekombinante Nucleotidsequenz gemäß Anspruch 11 enthält, wohingegen der (oder die) andere(n) rekombinante(n) Vektor(en) eine DNA-Sequenz enthält (enthalten), die für ein anderes Enzym als CCR, wie oben definiert, codiert.

18. Verfahren zur Erhöhung der Biosynthese von Lignin bei Pflanzen und somit zur Erhöhung der Gehalte an produziertem Lignin im Vergleich zu den normalen Gehalten an produziertem Lignin bei Pflanzen, **dadurch gekennzeichnet, dass** dies durch Transformation des Genoms dieser Pflanzen bewirkt wird, indem dort eingebracht wird:
- mindestens eine DNA-Sequenz gemäß einem der Ansprüche 2 und 3,
- und gegebenenfalls mindestens eine DNA-Sequenz, die für ein anderes Enzym
- als CCR codiert, das mit einem Ligninbiosyntheseschritt bei Pflanzen in Zusammenhang steht, insbesondere eine DNA-Sequenz, die für die CAD codiert,
wobei die Transformation durchgeführt wird mit:
- einem rekombinanten Vektor gemäß Anspruch 14, der eine rekombinante Nucleotidsequenz gemäß Anspruch 11 oder Anspruch 13 enthält,
- oder mehreren rekombinanten Vektoren, von denen mindestens einer eine rekombinante Nucleotidsequenz gemäß Anspruch 11 enthält, wohingegen der (oder die) andere(n) rekombinante(n) Vektor(en) eine DNA-Sequenz enthält (enthalten), die für ein anderes Enzym als CCR, wie oben definiert, codiert.

19. Pflanzen oder Teile von Pflanzen, insbesondere Zellen, Früchte, Samen, Pollen, die durch Einbringen mindestens einer Nucleotidsequenz gemäß einem der Ansprüche 2 bis 5 in ihr Genom transformiert sind.

20. Rekombinante CCR, dargestellt durch SEQ ID NO 2 oder SEQ ID NO 4, wie man sie durch Transformation von Pflanzenzellen durch Einbringen einer rekombinanten Nucleotidsequenz gemäß einem der Ansprüche 11 bis 13, insbesondere mit Hilfe eines Vektors gemäß Anspruch 14, in ihr Genom auf stabile Weise erhält.


### LISTE DE SEQUENCES

(1) INFORMATION GENERALE:
   (i) DEPOSANT:
      (A) NOM: CENTRE NATIONAL DE LA RECHERCHE SCIENTIFIQUE
      (B) RUE: 3, rue Michel-Ange
      (C) VILLE: PARIS 75016
      (E) PAYS: FRANCE
      (F) CODE POSTAL: F-75016
   (ii) TITRE DE L' INVENTION: SEQUENCES D'ADN CODANT POUR LA CINNAMOYI CoA REDUCTASE, ET SES APPLICATIONS DANS LE DOMAINE DE LA REGULATION DU TAUX DE LIGNINE CHEZ LES PLANTES
   (iii) NOMBRE DE SEQUENCES: 14
   (iv) FORME LISIBLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 1568 paires de bases
      (B) TYPE: acide nucléique
      (C) NOMBRE DE BRINS: double
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 278..1306
   (xi) DESCRIPTION DE LA SEQUENCE: SEQ ID NO: 1:
   CATGATTACG CCAAGCTCGA AATTAACCCT CACTAAAGGG AACAAAAGCT GGAGCTCCAC 60
   CGCGGTGGCG GCCGCTCTAG AACTAGTGGA TCCCCCGGGC TGCAGGAATT CGGCACGAGG 120
   GGATAGAGAA GAAAGGTGGT CATATTTCCC ACTTATTATT ACAAAGTAAC GTCACACCAA 180
   CTTTATCACC ACCTTTCTTC TCTATCCCAT TCATTCTCAT TCATTCATTC ACCTCACCTC     240
   ACCTCACCTC ACCTCCCTTT ACAAGAAGAA GGAATAT ATG CCT GCC GCT ACC GCC      295
                                            Met Pro Ala Ala Thr Ala
                                             1               5
   GCC GCC GCC GCC GAA TCT TCC TCA GTT TCC GGC GAA ACC ATA TGT GTC      343
   Ala Ala Ala Ala Glu Ser Ser Ser Val Ser Gly Glu Thr Ile Cys Val
               10                  15                  20
   ACC GGG GCC GGT GGC CTC ATC GCT TCT TGG ATG GTT AAG CTC CTC TTG      391
   Thr Gly Ala Gly Gly Leu Ile Ala Ser Trp Met Val Lys Leu Leu Leu
           25                  30                  35
   GAG AAA GGC TAT ACC GTT CGA GGA ACC TTG CGA AAC CCA GAT GAT CCA      439
   Glu Lys Gly Tyr Thr Val Arg Gly Thr Leu Arg Asn Pro Asp Asp Pro
       40                  45                  50
   AAA AAT GGG CAC TTG AAA AAG TTG GAA GGA GCA AAA GAA AGG CTA ACT      487
   Lys Asn Gly His Leu Lys Lys Leu Glu Gly Ala Lys Glu Arg Leu Thr
    55                  60                  65                  70
   TTG GTC AAA GTT GAT CTC CTT GAT CTT AAC TCC GTT AAA GAA GCT GTT      535
   Leu Val Lys Val Asp Leu Leu Asp Leu Asn Ser Val Lys Glu Ala Val
                   75                  80                  85
   AAT GGA TGT CAT GGT GTC TTT CAC ACT GCT TCT CCC GTT ACA GAT AAC      583
   Asn Gly Cys His Gly Val Phe His Thr Ala Ser Pro Val Thr Asp Asn
               90                  95                  100
   CCC GAG GAA ATG GTG GAG CCA GCA GTG AAT GGA GCA AAG AAT GTG ATC      631
   Pro Glu Glu Met Val Glu Pro Ala Val Asn Gly Ala Lys Asn Val Ile
           105                 110                 115
   ATA GCT GGT GCA GAA GCA AAA GTG AGG CGC GTG GTT TTC ACA TCA TCA      679
   Ile Ala Gly Ala Glu Ala Lys Val Arg Arg Val Val Phe Thr Ser Ser
       120                 125                 130
   ATT GGT GCA GTC TAT ATG GAC CCC AAT AGG AGT GTT GAT GTA GAG GTT      727
   Ile Gly Ala Val Tyr Met Asp Pro Asn Arg Ser Val Asp Val Glu Val
   135                 140                 145                 150
   GAT GAG TCT TGC TGG AGT GAT TTG GAG TTT TGC AAG AAA ACC AAG AAT      775
   Asp Glu Ser Cys Trp Ser Asp Leu Glu Phe Cys Lys Lys Thr Lys Asn
                   155                 160                 165
   TGG TAT TGC TAT GGG AAA GCA GTG GCA GAA GCA GCA GCA TGG GAT GTA      823
   Trp Tyr Cys Tyr Gly Lys Ala Val Ala Glu Ala Ala Ala Trp Asp Val
               170                 175                 180
   GCA AAA GAG AAA GGT GTG GAT TTG GTT GTA GTG AAT CCA GTT TTG GTT      871
   Ala Lys Glu Lys Gly Val Asp Leu Val Val Val Asn Pro Val Leu Val
           185                 190                 195
   CTT GGA CCA TTG CTA CAA CCT ACA ATC AAT GCA AGC ACA ATT CAC ATA      919
   Leu Gly Pro Leu Leu Gln Pro Thr Ile Asn Ala Ser Thr Ile His Ile
       200                 205                 210
   CTA AAA TAC CTA ACT GGT TCA GCT AAG ACC TAT GCA AAT GCA ACA CAA       967
   Leu Lys Tyr Leu Thr Gly Ser Ala Lys Thr Tyr Ala Asn Ala Thr Gln
   215                 220                 225                 230
   GCT TAT GTT CAT GTT AGG GAT GTT GCA TTA GCT CAC ATA CTT GTT TAT      1015
   Ala Tyr Val His Val Arg Asp Val Ala Leu Ala His Ile Leu Val Tyr
                   235                 240                 245
   GAG AAA CCT TCT GCT TCT GGT AGA TAC TTA TGT GCT GAA ACT TCA CTT      1063
   Glu Lys Pro Ser Ala Ser Gly Arg Tyr Leu Cys Ala Glu Thr Ser Leu
               250                 255                 260
   CAT CGT GGG GAG CTT GTT GAA ATT CTT GCT AAG TAT TTC CCT GAG TAC      1111
   His Arg Gly Glu Leu Val Glu Ile Leu Ala Lys Tyr Phe Pro Glu Tyr
           265                 270                 275
   CCA ATT CCT ACC AAG TGT TCA GAT GAA AAG AAT CCT CGA GTG AAA CCA      1159
   Pro Ile Pro Thr Lys Cys Ser Asp Glu Lys Asn Pro Arg Val Lys Pro
       280                 285                 290
   CAT ATC TTC TCA AAT AAA AAA CTG AAG GAT TTG GGA TTG GAA TTT ACA      1207
   His Ile Phe Ser Asn Lys Lys Leu Lys Asp Leu Gly Leu Glu Phe Thr
   295                 300                 305                 310
   CCA GTG AGT GAA TGT TTA TAT GAA ACC GTT AAG AGC CTA CAA GAC CAA      1255
   Pro Val Ser Glu Cys Leu Tyr Glu Thr Val Lys Ser Leu Gln Asp Gln
                   315                 320                 325
   GGT CAC CTT TCT ATT CCA AAC AAA GAA GAT TCT CTA GCA GTC AAA TCC      1303
   Gly His Leu Ser Ile Pro Asn Lys Glu Asp Ser Leu Ala Val Lys Ser
               330                 335                 340
   TAAACCAACC ATCCTTTGTT AACAAGTTCA ATTCAGGGCC AAAAAGAATC ATCTTTTAGC    1363
   TACCTGCGAG GCTTTAGGCT CTAGCAATTT GATACTATAA ATGACCGTAA TTGGATGGAT    1423
   AGTTGTAAGA AAGTATCATG CTAGAATTTA CTATTTGTCT TTATGTTTGA AAAATAAGCC    1483
   CATTATATTA AAAAAAAAAA AAAAAAAAAA AACTCGAGGG GGGGCCCGGT ACCCAATTCG    1543
   CCCTATAGTG AGTCGTATTA CAATT                                          1568
(2) INFORMATION POUR LA SEQ ID NO: 2:
   (i) CARACTERISTIQUES DE LA SEQUENCE:
      (A) LONGUEUR: 342 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 2:
   Met Pro Ala Ala Thr Ala Ala Ala Ala Ala Glu Ser Ser Ser Val Ser
   1 5 10 15
   Gly Glu Thr Ile Cys Val Thr Gly Ala Gly Gly Leu Ile Ala Ser Trp
   20 25 30
   Met Val Lys Leu Leu Leu Glu Lys Gly Tyr Thr Val Arg Gly Thr Leu
   35 40 45
   Arg Asn Pro Asp Asp Pro Lys Asn Gly His Leu Lys Lys Leu Glu Gly
   50 55 60
   Ala Lys Glu Arg Leu Thr Leu Val Lys Val Asp Leu Leu Asp Leu Asn
   65 70 75 80
   Ser Val Lys Glu Ala Val Asn Gly Cys His Gly Val Phe His Thr Ala
   85 90 95
   Ser Pro Val Thr Asp Asn Pro Glu Glu Met Val Glu Pro Ala Val Asn
   100 105 110
   Gly Ala Lys Asn Val Ile Ile Ala Gly Ala Glu Ala Lys Val Arg Arg
   115 120 125
   Val Val Phe Thr Ser Ser Ile Gly Ala Val Tyr Met Asp Pro Asn Arg
   130 135 140
   Ser Val Asp Val Glu Val Asp Glu Ser Cys Trp Ser Asp Leu Glu Phe
   145 150 155 160
   Cys Lys Lys Thr Lys Asn Trp Tyr Cys Tyr Gly Lys Ala Val Ala Glu
   165 170 175
   Ala Ala Ala Trp Asp Val Ala Lys Glu Lys Gly Val Asp Leu Val Val
   180 185 190
   Val Asn Pro Val Leu Val Leu Gly Pro Leu Leu Gln Pro Thr Ile Asn
   195 200 205
   Ala Ser Thr Ile His Ile Leu Lys Tyr Leu Thr Gly Ser Ala Lys Thr
   210 215 220
   Tyr Ala Asn Ala Thr Gln Ala Tyr Val His Val Arg Asp Val Ala Leu
   225 230 235 240
   Ala His Ile Leu Val Tyr Glu Lys Pro Ser Ala Ser Gly Arg Tyr Leu
   245 250 255
   Cys Ala Glu Thr Ser Leu His Arg Gly Glu Leu Val Glu Ile Leu Ala
   260 265 270
   Lys Tyr Phe Pro Glu Tyr Pro Ile Pro Thr Lys Cys Ser Asp Glu Lys
   275 280 285
   Asn Pro Arg Val Lys Pro His Ile Phe Ser Asn Lys Lys Leu Lys Asp
   290 295 300
   Leu Gly Leu Glu Phe Thr Pro Val Ser Glu Cys Leu Tyr Glu Thr Val
   305 310 315 320
   Lys Ser Leu Gln Asp Gln Gly His Leu Ser Ile Pro Asn Lys Glu Asp
                   325                 330                 335
   Ser Leu Ala Val Lys Ser
               340
(2) INFORMATION POUR LA SEQ ID NO: 3:
   (i) CARACTERISTIQUES DE LA SEQUENCE:
      (A) LONGUEUR: 1556 paires de bases
      (B) TYPE: acide nucléique
      (C) NOMBRE DE BRINS: double
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 195..1310
   (xi) DESCRIPTION DE LA SEQUENCE: SEQ ID NO: 3:
   GTTCCCATGA TTACGCCAAG CTCGAAATTA ACCCTCACTA AAGGGAACAA AAGCTGGAGC      60
   TCCACCGCGG TGGCGGCCGC TCTAGAACTA GTGGATCCCC CGGGCTGCAG GAATTCGGCA     120
   CGAGAGGACA CAAGCGAGCG CTAGCCAGAA GAGCAGCTGC AGGTACTATT ATCATCGTCG     180
   TCGTCGTCGC CAGG ATG ACC GTC GTC GAC GCC GTC GTC TCC TCC ACC GAT       230
                   Met Thr Val Val Asp Ala Val Val Ser Ser Thr Asp
                    1               5                  10
   GCC GGC GCC CCT GCC GCC GCC GCC GCA CCG GTA CCG GCG GGG AAC GGG       278
   Ala Gly Ala Pro Ala Ala Ala Ala Ala Pro Val Pro Ala Gly Asn Gly
           15                  20                  25
   CAG ACC GTG TGC GTC ACC GGC GCG GCC GGG TAC ATC GCC TCG TGG TTG       326
   Gln Thr Val Cys Val Thr Gly Ala Ala Gly Tyr Ile Ala Ser Trp Leu
       30                  35                  40
   GTG AAG CTG CTG CTC GAG AAG GGA TAC ACT GTG AAG GGC ACC GTG AGG       374
   Val Lys Leu Leu Leu Glu Lys Gly Tyr Thr Val Lys Gly Thr Val Arg
   45                  50                  55                  60
   AAC CCA GAT GAC CCG AAG AAC GCG CAC CTC AGG GCG CTG GAC GGC GCC       422
   Asn Pro Asp Asp Pro Lys Asn Ala His Leu Arg Ala Leu Asp Gly Ala
                   65                  70                  75
   GCC GAG CGG CTG ATC CTC TGC AAG GCC GAT CTG CTG GAC TAC GAC GCC       470
   Ala Glu Arg Leu Ile Leu Cys Lys Ala Asp Leu Leu Asp Tyr Asp Ala
               80                  85                  90
   ATC TGC CGC GCC GTG CAG GGC TGC CAG GGC GTC TTC CAC ACC GCC TCC       518
   Ile Cys Arg Ala Val Gln Gly Cys Gln Gly Val Phe His Thr Ala Ser
           95                  100                 105
   CCC GTC ACC GAC GAC CCG GAG CAA ATG GTG GAG CCG GCG GTG CGC GGC        566
   Pro Val Thr Asp Asp Pro Glu Gln Met Val Glu Pro Ala Val Arg Gly
       110                 115                 120
   ACC GAG TAC GTG ATC AAC GCG GCG GCG GAG GCC GGC ACG GTG CGG CGG        614
   Thr Glu Tyr Val Ile Asn Ala Ala Ala Glu Ala Gly Thr Val Arg Arg
   125                 130                 135                 140
   GTG GTG TTC ACG TCG TCC ATC GGC GCC GTG ACC ATG GAC CCC AAG CGC        662
   Val Val Phe Thr Ser Ser Ile Gly Ala Val Thr Met Asp Pro Lys Arg
                   145                 150                 155
   GGG CCC GAC GTC GTG GTC GAC GAG TCG TGC TGG AGC GAC CTC GAG TTC        710
   Gly Pro Asp Val Val Val Asp Glu Ser Cys Trp Ser Asp Leu Glu Phe
               160                 165                 170
   TGC GAG AAA ACC AGG AAC TGG TAC TGC TAC GGC AAG GCG GTG GCG GAG        758
   Cys Glu Lys Thr Arg Asn Trp Tyr Cys Tyr Gly Lys Ala Val Ala Glu
           175                 180                 185
   CAG GCG GCG TGG GAG GCG GCC CGG CGG CGG GGC GTG GAC CTG GTG GTG        806
   Gln Ala Ala Trp Glu Ala Ala Arg Arg Arg Gly Val Asp Leu Val Val
       190                 195                 200
   GTG AAC CCC GTG CTG GTG GTG GGC CCC CTG CTG CAG GCG ACG GTG AAC        854
   Val Asn Pro Val Leu Val Val Gly Pro Leu Leu Gln Ala Thr Val Asn
   205                 210                 215                 220
   GCC AGC ATC GCG CAC ATC CTC AAG TAC CTG GAC GGC TCG GCC CGC ACC        902
   Ala Ser Ile Ala His Ile Leu Lys Tyr Leu Asp Gly Ser Ala Arg Thr
                   225                 230                 235
   TTC GCC AAC GCC GTG CAG GCG TAC GTG GAC GTG CGC GAC GTG GCC GAC        950
   Phe Ala Asn Ala Val Gln Ala Tyr Val Asp Val Arg Asp Val Ala Asp
               240                 245                 250
   GCG CAC CTC CGC GTC TTC GAG AGC CCC CGC GCG TCC GGC CGC CAC CTC        998
   Ala His Leu Arg Val Phe Glu Ser Pro Arg Ala Ser Gly Arg His Leu
           255                 260                 265
   TGC GCC GAG CGC GTC CTC CAC CGC GAG GAC GTC GTC CGC ATC CTC GCC       1046
   Cys Ala Glu Arg Val Leu His Arg Glu Asp Val Val Arg Ile Leu Ala
       270                 275                 280
   AAG CTC TTC CCC GAG TAC CCC GTC CCA GCC AGG TGC TCC GAC GAG GTG       1094
   Lys Leu Phe Pro Glu Tyr Pro Val Pro Ala Arg Cys Ser Asp Glu Val
   285                 290                 295                 300
   AAT CCG CGG AAG CAG CCG TAC AAG TTC TCC AAC CAG AAG CTC CGG GAC       1142
   Asn Pro Arg Lys Gln Pro Tyr Lys Phe Ser Asn Gln Lys Leu Arg Asp
                   305                 310                 315
   CTG GGG CTG CAG TTC CGG CCG GTC AGC CAG TCG CTT TAC GAC ACG GTG       1190
   Leu Gly Leu Gln Phe Arg Pro Val Ser Gln Ser Leu Tyr Asp Thr Val
               320                 325                 330
   AAG AAC CTC CAG GAG AAG GGC CAC CTG CCG GTG CTC GGA GAG CGG ACG      1238
   Lys Asn Leu Gln Glu Lys Gly His Leu Pro Val Leu Gly Glu Arg Thr
           335                 340                 345
   ACG ACG GAG GCC GCC GAC AAG GAT GCC CCC GCG GCC GAG ATG CAG CAG      1286
   Thr Thr Glu Ala Ala Asp Lys Asp Ala Pro Ala Ala Glu Met Gln Gln
       350                 355                 360
   GGA GGG ATC GCC ATC CGT GCC TGAGAGGGCG ATGCCACACA TGAACACCAA         1337
   Gly Gly Ile Ala Ile Arg Ala
   365                 370
   AGCAATGTTC ATACTGCTGC CCTGCACCTG CACCTTCCCC TGCTGTGTAA ACAGGCCTGT    1397
   GTTTGTTCTG GCTGATAGTG ATGTACCCTA AGACTTGTAA CGTCATGTTC GTTCTTGTGA    1457
   ACTATAGCGA GTGAATAAAA TTGGTTAATG TTGGATAATT CCAAAAAAAA AAAAAAAAAA    1517
   CTCGAGGGGG GGCCCGGTAC CCAATTCGCC CTATAGTGA                          1556
(2) INFORMATION POUR LA SEQ ID NO: 4:
   (i) CARACTERISTIQUES DE LA SEQUENCE:
      (A) LONGUEUR: 371 acides aminés
      (B) TYPE: acide aminé
      (D) CONFIGURATION: linéaire
   (ii) TYPE DE MOLECULE: protéine
   (xi) DESCRIPTION DE LA SEQUENCE: SEO ID NO: 4:
   Met Thr Val Val Asp Ala Val Val Ser Ser Thr Asp Ala Gly Ala Pro
   1 5 10 15
   Ala Ala Ala Ala Ala Pro Val Pro Ala Gly Asn Gly Gln Thr Val Cys
   20 25 30
   Val Thr Gly Ala Ala Gly Tyr Ile Ala Ser Trp Leu Val Lys Leu Leu
   35 40 45
   Leu Glu Lys Gly Tyr Thr Val Lys Gly Thr Val Arg Asn Pro Asp Asp
   50 55 60
   Pro Lys Asn Ala His Leu Arg Ala Leu Asp Gly Ala Ala Glu Arg Leu
   65 70 75 80
   Ile Leu Cys Lys Ala Asp Leu Leu Asp Tyr Asp Ala Ile Cys Arg Ala
   85 90 95
   Val Gln Gly Cys Gln Gly Val Phe His Thr Ala Ser Pro Val Thr Asp
   100 105 110
   Asp Pro Glu Gln Met Val Glu Pro Ala Val Arg Gly Thr Glu Tyr Val
   115 120 125
   Ile Asn Ala Ala Ala Glu Ala Gly Thr Val Arg Arg Val Val Phe Thr
   130 135 140
   Ser Ser Ile Gly Ala Val Thr Met Asp Pro Lys Arg Gly Pro Asp Val
   145                 150                 155                 160
   Val Val Asp Glu Ser Cys Trp Ser Asp Leu Glu Phe Cys Glu Lys Thr
                   165                 170                 175
   Arg Asn Trp Tyr Cys Tyr Gly Lys Ala Val Ala Glu Gln Ala Ala Trp
               180                 185                 190
   Glu Ala Ala Arg Arg Arg Gly Val Asp Leu Val Val Val Asn Pro Val
           195                 200                 205
   Leu Val Val Gly Pro Leu Leu Gln Ala Thr Val Asn Ala Ser Ile Ala
       210                 215                 220
   His Ile Leu Lys Tyr Leu Asp Gly Ser Ala Arg Thr Phe Ala Asn Ala
   225                 230                 235                 240
   Val Gln Ala Tyr Val Asp Val Arg Asp Val Ala Asp Ala His Leu Arg
                   245                 250                 255
   Val Phe Glu Ser Pro Arg Ala Ser Gly Arg His Leu Cys Ala Glu Arg
               260                 265                 270
   Val Leu His Arg Glu Asp Val Val Arg Ile Leu Ala Lys Leu Phe Pro
           275                 280                 285
   Glu Tyr Pro Val Pro Ala Arg Cys Ser Asp Glu Val Asn Pro Arg Lys
       290                 295                 300
   Gln Pro Tyr Lys Phe Ser Asn Gln Lys Leu Arg Asp Leu Gly Leu Gln
   305                 310                 315                 320
   Phe Arg Pro Val Ser Gln Ser Leu Tyr Asp Thr Val Lys Asn Leu Gln
                   325                 330                 335
   Glu Lys Gly His Leu Pro Val Leu Gly Glu Arg Thr Thr Thr Glu Ala
               340                 345                 350
   Ala Asp Lys Asp Ala Pro Ala Ala Glu Met Gln Gln Gly Gly Ile Ala
           355                 360                 365
   Ile Arg Ala
       370
(2) INFORMATION POUR LA SEQ ID NO: 5:
   (i) CARACTERISTIQUES DE LA SEQUENCE:
      (A) LONGUEUR: 1297 paires de bases
      (B) TYPE: acide nucléique
      (C) NOMBRE DE BRINS: double
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 136..1140
   (xi) DESCRIPTION DE LA SEQUENCE: SEQ ID NO: 5:
   CGGCCGGGAC GACCCGTTCC TCTTCTTCCG GGTCACCGTC ACCATGTTAC ACAACATCTC      60
   CGGCTAAAAA AAAAAGGAAA AAAAGCGCAA CCTCCACCTC CTGAACCCCT CTCCCCCCTC     120
   GCCGGCAATC CCACC ATG CCC GTC GAC GCC CTC CCC GGT TCC GGC CAG ACC     171
                    Met Pro Val Asp Ala Leu Pro Gly Ser Gly Gln Thr
                     1               5                  10
   GTC TGC GTC ACC GGC GCC GGC GGG TTC ATC GCC TCC TGG ATT GTC AAG      219
   Val Cys Val Thr Gly Ala Gly Gly Phe Ile Ala Ser Trp Ile Val Lys
            15                  20                  25
   CTT CTC CTC GAG CGA GGC TAC ACC GTG CGA GGA ACC GTC AGG AAC CCA      267
   Leu Leu Leu Glu Arg Gly Tyr Thr Val Arg Gly Thr Val Arg Asn Pro
        30                  35                  40
   GAC GAC CCG AAG AAT GGT CAT CTG AGA GAT CTG GAA GGA GCC AGC GAG      315
   Asp Asp Pro Lys Asn Gly His Leu Arg Asp Leu Glu Gly Ala Ser Glu
    45                  50                  55                  60
   AGG CTG ACG CTG TAC AAG GGT GAT CTG ATG GAC TAC GGG AGC TTG GAA      363
   Arg Leu Thr Leu Tyr Lys Gly Asp Leu Met Asp Tyr Gly Ser Leu Glu
                    65                  70                  75
   GAA GCC ATC AAG GGG TGC GAC GGC GTC GTC CAC ACC GCC TCT CCG GTC      411
   Glu Ala Ile Lys Gly Cys Asp Gly Val Val His Thr Ala Ser Pro Val
                80                  85                  90
   ACC GAC GAT CCT GAG CAA ATG GTG GAG CCA GCG GTG ATC GGG ACG AAA      459
   Thr Asp Asp Pro Glu Gln Met Val Glu Pro Ala Val Ile Gly Thr Lys
            95                 100                 105
   AAT GTG ATC GTC GCA GCG GCG GAG GCC AAG GTC CGG CGG GTT GTG TTC 507
   Asn Val Ile Val Ala Ala Ala Glu Ala Lys Val Arg Arg Val Val Phe
   110 115 120
   ACC TCC TCC ATC GGT GCA GTC ACC ATG GAC CCC AAC CGG GCA GAC GTT 555
   Thr Ser Ser Ile Gly Ala Val Thr Met Asp Pro Asn Arg Ala Asp Val
   125 130 135 140
   GTG GTG GAC GAG TCT TGT TGG AGC GAC CTC GAA TTT TGC AAG AGC ACT 603
   Val Val Asp Glu Ser Cys Trp Ser Asp Leu Glu Phe Cys Lys Ser Thr
   145 150 155
   AAG AAC TGG TAT TGC TAC GGC AAG GCA GTG GCG GAG AAG GCC GCT TGG 651
   Lys Asn Trp Tyr Cys Tyr Gly Lys Ala Val Ala Glu Lys Ala Ala Trp
   160 165 170
   CCA GAG GGC AAG GAG AGA GGG GTT GAC CTC GTG GTG ATT AAC CCT GTG 699
   Pro Glu Gly Lys Glu Arg Gly Val Asp Leu Val Val Ile Asn Pro Val
   175 180 185
   CTC GTG CTT GGA CCG CTC CTT CAG TCG ACG ATC AAT GCG AGC ATC ATC 747
   Leu Val Leu Gly Pro Leu Leu Gln Ser Thr Ile Asn Ala Ser Ile Ile
   190 195 200
   CAC ATC CTC AAG TAC TTG ACT GGC TCA GCC AAG ACC TAC GCC AAC TCG 795
   His Ile Leu Lys Tyr Leu Thr Gly Ser Ala Lys Thr Tyr Ala Asn Ser
   205 210 215 220
   GTC CAG GCG TAC GTG CAC GTC AAG GAC GTC GCG CTT GCC CAC GTC CTT 843
   Val Gln Ala Tyr Val His Val Lys Asp Val Ala Leu Ala His Val Leu
   225 230 235
   GTC TTG GAG ACC CCA TCC GCC TCA GGC CGC TAT TTG TGC GCC GAG AGC 891
   Val Leu Glu Thr Pro Ser Ala Ser Gly Arg Tyr Leu Cys Ala Glu Ser
   240 245 250
   GTC CTC CAC CGT GGC GAT GTG GTG GAA ATC CTT GCC AAG TTC TTC CCT 939
   Val Leu His Arg Gly Asp Val Val Glu Ile Leu Ala Lys Phe Phe Pro
   255 260 265
   GAG TAT AAT GTA CCG ACC AAG TGC TCT GAT GAG GTG AAC CCA AGA GTA         987
   Glu Tyr Asn Val Pro Thr Lys Cys Ser Asp Glu Val Asn Pro Arg Val
       270                 275                 280
   AAA CCA TAC AAG TTC TCC AAC CAG AAG CTG AGA GAC TTG GGG CTC GAG        1035
   Lys Pro Tyr Lys Phe Ser Asn Gln Lys Leu Arg Asp Leu Gly Leu Glu
   285                 290                 295                 300
   TTC ACC CCG GTG AAG CAG TGC CTG TAC GAA ACT GTC AAG AGC TTG CAG        1083
   Phe Thr Pro Val Lys Gln Cys Leu Tyr Glu Thr Val Lys Ser Leu Gln
                   305                 310                 315
   GAG AAA GGC CAC CTA CCA GTC CCC TCC CCG CCG GAA GAT TCG GTG CGT        1131
   Glu Lys Gly His Leu Pro Val Pro Ser Pro Pro Glu Asp Ser Val Arg
               320                 325                 330
   ATT CAG GGA TGATCTTAGA TCCATCACGG TGCGCATTTG TAATCCGGAG                1180
   Ile Gln Gly
           335
   AAATGAGAGA AACATGTGGG AATTTGTTTG TACTTTTCTA AGTCAAACCT GGAGATACCA      1240
   ACCCTGAGTT CTGCATTGGA ATGGAAGTTG TCAATTGTTC CAAAAAAAAA AAAAAAA         1297
(2) INFORMATION POUR LA SEQ ID NO: 6:
   (i) CARACTERISTIQUES DE LA SEQUENCE:
      (A) LONGUEUR: 335 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 6:
   Met Pro Val Asp Ala Leu Pro Gly Ser Gly Gln Thr Val Cys Val Thr
   1               5                   10                  15
   Gly Ala Gly Gly Phe Ile Ala Ser Trp Ile Val Lys Leu Leu Leu Glu
   20 25 30
   Arg Gly Tyr Thr Val Arg Gly Thr Val Arg Asn Pro Asp Asp Pro Lys
   35 40 45
   Asn Gly His Leu Arg Asp Leu Glu Gly Ala Ser Glu Arg Leu Thr Leu
   50 55 60
   Tyr Lys Gly Asp Leu Met Asp Tyr Gly Ser Leu Glu Glu Ala Ile Lys
   65 70 75 80
   Gly Cys Asp Gly Val Val His Thr Ala Ser Pro Val Thr Asp Asp Pro
   85 90 95
   Glu Gln Met Val Glu Pro Ala Val Ile Gly Thr Lys Asn Val Ile Val
   100 105 110
   Ala Ala Ala Glu Ala Lys Val Arg Arg Val Val Phe Thr Ser Ser Ile
   115 120 125
   Gly Ala Val Thr Met Asp Pro Asn Arg Ala Asp Val Val Val Asp Glu
   130 135 140
   Ser Cys Trp Ser Asp Leu Glu Phe Cys Lys Ser Thr Lys Asn Trp Tyr
   145 150 155 160
   Cys Tyr Gly Lys Ala Val Ala Glu Lys Ala Ala Trp Pro Glu Gly Lys
   165 170 175
   Glu Arg Gly Val Asp Leu Val Val Ile Asn Pro Val Leu Val Leu Gly
   180 185 190
   Pro Leu Leu Gln Ser Thr Ile Asn Ala Ser Ile Ile His Ile Leu Lys
   195 200 205
   Tyr Leu Thr Gly Ser Ala Lys Thr Tyr Ala Asn Ser Val Gln Ala Tyr
   210 215 220
   Val His Val Lys Asp Val Ala Leu Ala His Val Leu Val Leu Glu Thr
   225                 230                 235                 240
   Pro Ser Ala Ser Gly Arg Tyr Leu Cys Ala Glu Ser Val Leu His Arg
                   245                 250                 255
   Gly Asp Val Val Glu Ile Leu Ala Lys Phe Phe Pro Glu Tyr Asn Val
               260                 265                 270
   Pro Thr Lys Cys Ser Asp Glu Val Asn Pro Arg Val Lys Pro Tyr Lys
           275                 280                 285
   Phe Ser Asn Gln Lys Leu Arg Asp Leu Gly Leu Glu Phe Thr Pro Val
       290                 295                 300
   Lys Gln Cys Leu Tyr Glu Thr Val Lys Ser Leu Gln Glu Lys Gly His
   305                 310                 315                 320
   Leu Pro Val Pro Ser Pro Pro Glu Asp Ser Val Arg Ile Gln Gly
                   325                 330                 335
(2) INFORMATION POUR LA SEQ ID NO: 7:
   (i) CARACTERISTIQUES DE LA SEQUENCE:
      (A) LONGUEUR: 1376 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 99..1112
   (xi) DESCRIPTION DE LA SEQUENCE: SEQ ID NO: 7:
   GAAAACACAC CTCCTCTCTT CTTTGTCTCT GTCTGTTCTC CACTTTCCCA GTCACCAAAC      60
   TCGTATGCAT ATAATTACAT TTATCTAAAT ATAACAAC ATG CCT GTT GAT GCT      113
                                             Met Pro Val Asp Ala
                                              1               5
   TCA TCA CTT TCA GGC CAA GGC CAA ACT ATC TGT GTC ACC GGG GGT GGT    161
   Ser Ser Leu Ser Gly Gln Gly Gln Thr Ile Cys Val Thr Gly Gly Gly
                    10                  15                  20
   GGT TTC ATT GCT TCT TGG ATG GTT AAA CTT CTT TTA GAT AAA GGT TAC    209
   Gly Phe Ile Ala Ser Trp Met Val Lys Leu Leu Leu Asp Lys Gly Tyr
                25                  30                  35
   ACT GTT AGA GGA ACT GCG AGG AAC CCA GCT GAT CCC AAG AAT TCT CAT    257
   Thr Val Arg Gly Thr Ala Arg Asn Pro Ala Asp Pro Lys Asn Ser His
            40                  45                  50
   TTG AGG GAG CTT GAA GGA GCT GAA GAA AGA TTA ACT TTA TGC AAA GCT    305
   Leu Arg Glu Leu Glu Gly Ala Glu Glu Arg Leu Thr Leu Cys Lys Ala
        55                  60                  65
   GAT CTT CTT GAT TAT GAG TCT CTT AAA GAG GGT ATT CAA GGG TGT GAT    353
   Asp Leu Leu Asp Tyr Glu Ser Leu Lys Glu Gly Ile Gln Gly Cys Asp
    70                  75                  80                  85
   GGT GTT TTC CAC ACT GCT TCT CCT GTC ACA GAT GAT CCG GAA GAA ATG    401
   Gly Val Phe His Thr Ala Ser Pro Val Thr Asp Asp Pro Glu Glu Met
                    90                  95                 100
   GTG GAG CCA GCA GTG AAC GGG ACC AAA AAT GTG ATA ATT GCG GCG GCT    449
   Val Glu Pro Ala Val Asn Gly Thr Lys Asn Val Ile Ile Ala Ala Ala
               105                 110                 115
   GAG GCC AAA GTC CGA CGA GTG GTG TTC ACG TCA TCA ATT GGC GCT GTG    497
   Glu Ala Lys Val Arg Arg Val Val Phe Thr Ser Ser Ile Gly Ala Val
           120                 125                 130
   TAC ATG GAT CCC AAT AAG GGC CCA GAT GTT GTC ATT GAT GAG TCT TGC    545
   Tyr Met Asp Pro Asn Lys Gly Pro Asp Val Val Ile Asp Glu Ser Cys
       135                 140                 145
   TGG AGT GAT CTT GAA TTC TGC AAG AAC ACC AAG AAT TGG TAT TGC TAT 593
   Trp Ser Asp Leu Glu Phe Cys Lys Asn Thr Lys Asn Trp Tyr Cys Tyr
   150 155 160 165
   GGA AAG GCT GTG GCA GAA CAA GCT GCA TGG GAT ATG GCT AAG GAG AAA 641
   Gly Lys Ala Val Ala Glu Gln Ala Ala Trp Asp Met Ala Lys Glu Lys
   170 175 180
   GGG GTG GAC CTA GTG GTG GTT AAC CCA GTG CTG GTG CTT GGA CCA TTG 689
   Gly Val Asp Leu Val Val Val Asn Pro Val Leu Val Leu Gly Pro Leu
   185 190 195
   TTG CAG CCC ACT GTC AAT GCT AGC ATC ACT CAC ATC CTC AAG TAC CTC 737
   Leu Gln Pro Thr Val Asn Ala Ser Ile Thr His Ile Leu Lys Tyr Leu
   200 205 210
   ACC GGC TCA GCC AAG ACA TAT GCT AAC TCT GTT CAA GCT TAT GTG CAT 785
   Thr Gly Ser Ala Lys Thr Tyr Ala Asn Ser Val Gln Ala Tyr Val His
   215 220 225
   GTT AGG GAT GTG GCA CTA GCC CAC ATT TTA GTC TTT GAG ACG CCT TCC 833
   Val Arg Asp Val Ala Leu Ala His Ile Leu Val Phe Glu Thr Pro Ser
   230 235 240 245
   GCC TCC GGC CGT TAC CTT TGC TCT GAG AGC GTT CTC CAC CGT GGA GAG 881
   Ala Ser Gly Arg Tyr Leu Cys Ser Glu Ser Val Leu His Arg Gly Glu
   250 255 260
   GTG GTG GAA ATC CTT GCA AAG TTC TTC CCT GAG TAC CCC ATC CCT ACC 929
   Val Val Glu Ile Leu Ala Lys Phe Phe Pro Glu Tyr Pro Ile Pro Thr
   265 270 275
   AAG TGC TCA GAT GAG AAG AAC CCA AGA AAA CAA CCT TAC AAG TTC TCA 977
   Lys Cys Ser Asp Glu Lys Asn Pro Arg Lys Gln Pro Tyr Lys Phe Ser
   280 285 290
   AAC CAG AAG CTA AGG GAT CTG GGT TTC GAA TTC ACC CCA GTA AAG CAG 1025
   Asn Gln Lys Leu Arg Asp Leu Gly Phe Glu Phe Thr Pro Val Lys Gln
   295 300 305
   TGT CTG TAT GAA ACT GTT AAG AGT TTG CAG GAA AAG GGT CAC CTT CCA 1073
   Cys Leu Tyr Glu Thr Val Lys Ser Leu Gln Glu Lys Gly His Leu Pro
   310 315 320 325
   ATC CCA AAA CAA GCT GCA GAA GAG TCT TTG AAA ATT CAA TAAGGCCTCT 1122
   Ile Pro Lys Gln Ala Ala Glu Glu Ser Leu Lys Ile Gln
   330 335
   TGGAACTATT TATTAGGATT GTTCCATACC CCAAGTTTGG ATCGCAAATG CTAGGGAAAG 1182
   GAGCATATTA AAGAATGCCA ATGTGCAGGT GTTTTAGTAT TTTACATGAA GAACTCTGAT 1242
   TATCCTTGTG CTTATAATAA TTTTTTTCAA GTGAGTGTCT TCAAATGTTC AACTTGTATT 1302
   TGTGGTTGTC TAACTTTATC CAGTTTCAAT ATAAAAGAGG AACGATTCTA TGTCTTAAAA 1362
   AAAAAAAAAA AAAA 1376
(2) INFORMATION POUR LA SEQ ID NO: 8:
   (i) CARACTERISTIQUES DE LA SEQUENCE:
      (A) LONGUEUR: 338 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 8:
   Met Pro Val Asp Ala Ser Ser Leu Ser Gly Gln Gly Gln Thr Ile Cys
   1 5 10 15
   Val Thr Gly Gly Gly Gly Phe Ile Ala Ser Trp Met Val Lys Leu Leu
   20 25 30
   Leu Asp Lys Gly Tyr Thr Val Arg Gly Thr Ala Arg Asn Pro Ala Asp
   35 40 45
   Pro Lys Asn Ser His Leu Arg Glu Leu Glu Gly Ala Glu Glu Arg Leu
   50 55 60
   Thr Leu Cys Lys Ala Asp Leu Leu Asp Tyr Glu Ser Leu Lys Glu Gly
   65 70 75 80
   Ile Gln Gly Cys Asp Gly Val Phe His Thr Ala Ser Pro Val Thr Asp
   85 90 95
   Asp Pro Glu Glu Met Val Glu Pro Ala Val Asn Gly Thr Lys Asn Val
   100 105 110
   Ile Ile Ala Ala Ala Glu Ala Lys Val Arg Arg Val Val Phe Thr Ser
   115 120 125
   Ser Ile Gly Ala Val Tyr Met Asp Pro Asn Lys Gly Pro Asp Val Val
   130 135 140
   Ile Asp Glu Ser Cys Trp Ser Asp Leu Glu Phe Cys Lys Asn Thr Lys
   145 150 155 160
   Asn Trp Tyr Cys Tyr Gly Lys Ala Val Ala Glu Gln Ala Ala Trp Asp
   165 170 175
   Met Ala Lys Glu Lys Gly Val Asp Leu Val Val Val Asn Pro Val Leu
   180 185 190
   Val Leu Gly Pro Leu Leu Gln Pro Thr Val Asn Ala Ser Ile Thr His
   195 200 205
   Ile Leu Lys Tyr Leu Thr Gly Ser Ala Lys Thr Tyr Ala Asn Ser Val
   210 215 220
   Gln Ala Tyr Val His Val Arg Asp Val Ala Leu Ala His Ile Leu Val
   225 230 235 240
   Phe Glu Thr Pro Ser Ala Ser Gly Arg Tyr Leu Cys Ser Glu Ser Val
   245 250 255
   Leu His Arg Gly Glu Val Val Glu Ile Leu Ala Lys Phe Phe Pro Glu
               260                 265                 270
   Tyr Pro Ile Pro Thr Lys Cys Ser Asp Glu Lys Asn Pro Arg Lys Gln
           275                 280                 285
   Pro Tyr Lys Phe Ser Asn Gln Lys Leu Arg Asp Leu Gly Phe Glu Phe
       290                 295                 300
   Thr Pro Val Lys Gln Cys Leu Tyr Glu Thr Val Lys Ser Leu Gln Glu
   305                 310                 315                 320
   Lys Gly His Leu Pro Ile Pro Lys Gln Ala Ala Glu Glu Ser Leu Lys
                   325                 330                 335
   Ile Gln
(2) INFORMATION POUR LA SEQ ID NO: 9:
   (i) CARACTERISTIQUES DE LA SEQUENCE:
      (A) LONGUEUR: 1273 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 66..1091
   (xi) DESCRIPTION DE LA SEQUENCE: SEQ ID NO: 9:
   TCGTAGCTCT TCCCTTTCAC CAACAAGCTA GTTTAGACAA GTACAGTGGT ACTGTAAGAG        60
   CAACA ATG ACC GTT GTC GAC GCC GCC GCG CCG CAG CTG CCT GGC CAT           107
         Met Thr Val Val Asp Ala Ala Ala Pro Gln Leu Pro Gly His
           1               5                  10
   GGG CAG ACC GTG TGC GTC ACC GGC GCC GCG GGG TAC ATC GCG TCG GGG 155
   Gly Gln Thr Val Cys Val Thr Gly Ala Ala Gly Tyr Ile Ala Ser Gly
   15 20 25 30
   CTC GTC AAG CTG CTC CTG GAG AGA GGC TAC ACC GTG AAG GGC ACA GTG 203
   Leu Val Lys Leu Leu Leu Glu Arg Gly Tyr Thr Val Lys Gly Thr Val
   35 40 45
   AGG AAC CCA GAT GAT CCC AAG AAC GCC CAC CTG AAG GCG CTG GAC GGC 251
   Arg Asn Pro Asp Asp Pro Lys Asn Ala His Leu Lys Ala Leu Asp Gly
   50 55 60
   GCC ACC AAG AGG CTG ATC CTC TGC AAA GCC GAC CTC CTC GAC TAC GAC 299
   Ala Thr Lys Arg Leu Ile Leu Cys Lys Ala Asp Leu Leu Asp Tyr Asp
   65 70 75
   GCC ATA TGC GCC GCC GTC GAG GGC TGC CAC GGC GTG TTC CAC ACC GCC 347
   Ala Ile Cys Ala Ala Val Glu Gly Cys His Gly Val Phe His Thr Ala
   80 85 90
   TCT CCA GTC ACC GAT GAT CCT GAG CAG ATG GTG GAG CCG GCG GTG CGG 395
   Ser Pro Val Thr Asp Asp Pro Glu Gln Met Val Glu Pro Ala Val Arg
   95 100 105 110
   GGC ACG GAG TAC GTG ATC AAC GCG GCA GCG GAT GCG GGA ACG GTG CGC 443
   Gly Thr Glu Tyr Val Ile Asn Ala Ala Ala Asp Ala Gly Thr Val Arg
   115 120 125
   CGG GTG GTG TTC ACG TCG TCA ATC GGT GCC ATC ACC ATG GAC CCC AAC 491
   Arg Val Val Phe Thr Ser Ser Ile Gly Ala Ile Thr Met Asp Pro Asn
   130 135 140
   CGC GGT CCT GAC GTA GTC GTC AAT GAG TCC TGC TGG AGC GAC CTC GAA 539
   Arg Gly Pro Asp Val Val Val Asn Glu Ser Cys Trp Ser Asp Leu Glu
   145 150 155
   TTC TGC AAG AAA ACC AAG AAC TGG TAC TGC TAC GGC AAG GCC GTG GCG 587
   Phe Cys Lys Lys Thr Lys Asn Trp Tyr Cys Tyr Gly Lys Ala Val Ala
   160 165 170
   GAG CAG GCT GCG TGG GAG GCG GCC AGG AAG CGC GGC ATC GAC CTC GTC 635
   Glu Gln Ala Ala Trp Glu Ala Ala Arg Lys Arg Gly Ile Asp Leu Val
   175 180 185 190
   GTC GTG AAC CCT GTG CTC GTG GTA GGG CCG CTG CTG CAA CCA ACG GTG 683
   Val Val Asn Pro Val Leu Val Val Gly Pro Leu Leu Gln Pro Thr Val
   195 200 205
   AAC GCT AGC GCC GCA CAC ATC CTC AAG TAC CTC GAC GGC TCG GCC AAG 731
   Asn Ala Ser Ala Ala His Ile Leu Lys Tyr Leu Asp Gly Ser Ala Lys
   210 215 220
   AAG TAC GCC AAC GCT GTG CAG TCA TAC GTA GAC GTG CGT GAC GTA GCC 779
   Lys Tyr Ala Asn Ala Val Gln Ser Tyr Val Asp Val Arg Asp Val Ala
   225 230 235
   GGC GCG CAC ATC CGG GTG TTC GAG GCG CCT GAG GCG TCG GGC CGG TAC 827
   Gly Ala His Ile Arg Val Phe Glu Ala Pro Glu Ala Ser Gly Arg Tyr
   240 245 250
   CTC TGC GCC GAG CGC GTG CTG CAC CGT GGG GAC GTT GTC CAA ATC CTC 875
   Leu Cys Ala Glu Arg Val Leu His Arg Gly Asp Val Val Gln Ile Leu
   255 260 265 270
   AGC AAA CTC TTG CCT GAG TAC CCT GTG CCA ACA AGG TGC TCT GAT GAA 923
   Ser Lys Leu Leu Pro Glu Tyr Pro Val Pro Thr Arg Cys Ser Asp Glu
   275 280 285
   GTG AAC CCA CGG AAG CAG CCT TAT AAG ATG TCC AAC CAG AAG CTG CAG 971
   Val Asn Pro Arg Lys Gln Pro Tyr Lys Met Ser Asn Gln Lys Leu Gln
   290 295 300
   GAT CTT GGC CTC CAG TTC ACT CCT GTG AAC GAC TCT CTG TAT GAG ACC 1019
   Asp Leu Gly Leu Gln Phe Thr Pro Val Asn Asp Ser Leu Tyr Glu Thr
   305 310 315
   GTG AAG AGC CTC CAG GAG AAG GGA CAT CTC CTA GTA CCA AGC AAA CCC 1067
   Val Lys Ser Leu Gln Glu Lys Gly His Leu Leu Val Pro Ser Lys Pro
   320 325 330
   GAG GGA TTA AAC GGT GTA ACG GCA TGATACTGCT AAAGAAGCAG CAGAGTTCAC 1121
   Glu Gly Leu Asn Gly Val Thr Ala
   335 340
   GTGCTCCTGT AACATGGTCA AACATGAGTT GTTTTTCTGT ATAAATTCTA TCCAGTATCG 1181
   TGTTATTTAA GTGAACTAAG AGAACAGAAT ATTGTATCAT CTTCGATGTC CAATACCTGG 1241
   AAGTGATTTG TTTTGCCACC TAAAAAAAAA AA 1273
(2) INFORMATION POUR LA SEQ ID NO: 10:
   (i) CARACTERISTIQUES DE LA SEQUENCE:
      (A) LONGUEUR: 342 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 10:
   Met Thr Val Val Asp Ala Ala Ala Pro Gln Leu Pro Gly His Gly Gln
   1 5 10 15
   Thr Val Cys Val Thr Gly Ala Ala Gly Tyr Ile Ala Ser Gly Leu Val
   20 25 30
   Lys Leu Leu Leu Glu Arg Gly Tyr Thr Val Lys Gly Thr Val Arg Asn
   35 40 45
   Pro Asp Asp Pro Lys Asn Ala His Leu Lys Ala Leu Asp Gly Ala Thr
   50 55 60
   Lys Arg Leu Ile Leu Cys Lys Ala Asp Leu Leu Asp Tyr Asp Ala Ile
   65 70 75 80
   Cys Ala Ala Val Glu Gly Cys His Gly Val Phe His Thr Ala Ser Pro
   85 90 95
   Val Thr Asp Asp Pro Glu Gln Met Val Glu Pro Ala Val Arg Gly Thr
   100 105 110
   Glu Tyr Val Ile Asn Ala Ala Ala Asp Ala Gly Thr Val Arg Arg Val
   115 120 125
   Val Phe Thr Ser Ser Ile Gly Ala Ile Thr Met Asp Pro Asn Arg Gly
   130 135 140
   Pro Asp Val Val Val Asn Glu Ser Cys Trp Ser Asp Leu Glu Phe Cys
   145 150 155 160
   Lys Lys Thr Lys Asn Trp Tyr Cys Tyr Gly Lys Ala Val Ala Glu Gln
   165 170 175
   Ala Ala Trp Glu Ala Ala Arg Lys Arg Gly Ile Asp Leu Val Val Val
   180 185 190
   Asn Pro Val Leu Val Val Gly Pro Leu Leu Gln Pro Thr Val Asn Ala
   195 200 205
   Ser Ala Ala His Ile Leu Lys Tyr Leu Asp Gly Ser Ala Lys Lys Tyr
   210 215 220
   Ala Asn Ala Val Gln Ser Tyr Val Asp Val Arg Asp Val Ala Gly Ala
   225 230 235 240
   His Ile Arg Val Phe Glu Ala Pro Glu Ala Ser Gly Arg Tyr Leu Cys
   245 250 255
   Ala Glu Arg Val Leu His Arg Gly Asp Val Val Gln Ile Leu Ser Lys
   260 265 270
   Leu Leu Pro Glu Tyr Pro Val Pro Thr Arg Cys Ser Asp Glu Val Asn
   275 280 285
   Pro Arg Lys Gln Pro Tyr Lys Met Ser Asn Gln Lys Leu Gln Asp Leu
   290 295 300
   Gly Leu Gln Phe Thr Pro Val Asn Asp Ser Leu Tyr Glu Thr Val Lys
   305 310 315 320
   Ser Leu Gln Glu Lys Gly His Leu Leu Val Pro Ser Lys Pro Glu Gly
   325 330 335
   Leu Asn Gly Val Thr Ala
   340
(2) INFORMATION POUR LA SEQ ID NO: 11:
   (i) CARACTERISTIQUES DE LA SEQUENCE:
      (A) LONGUEUR: 1293 paires de bases
      (B) TYPE: acide nucléique
      (C) NOMBRE DE BRINS: simple
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 95..1108
   (xi) DESCRIPTION DE LA SEQUENCE: SEQ ID NO: 11:
   CCGAGCCTAT TTCTTCCCTA TATCCACTCA TCCTTGTCTT ATATCATCAT CATCATCATC 60
   TACCTAAACC TGAGCTCAAC AGAAAAGTAA TACC ATG CCG TCA GTT TCC GGC 112
   Met Pro Ser Val Ser Gly
   1 5
   CAA ATC GTT TGT GTT ACT GGC GCC GGA GGT TTC ATC GCC TCT TGG CTC 160
   Gln Ile Val Cys Val Thr Gly Ala Gly Gly Phe Ile Ala Ser Trp Leu
   10 15 20
   GTT AAA ATT CTT CTG GAA AAA GGC TAC ACT GTT AGA GGA ACA GTA CGA 208
   Val Lys Ile Leu Leu Glu Lys Gly Tyr Thr Val Arg Gly Thr Val Arg
   25 30 35
   AAT CCA GAT GAT CGA AAA AAT AGT CAT TTG AGG GAG CTT GAA CGA GCA      256
   Asn Pro Asp Asp Arg Lys Asn Ser His Leu Arg Glu Leu Glu Arg Ala
        40                  45                  50
   AAA GAG ACA TTG ACT CTG TGC AGA GCT GAT CTT CTT GAT TTT CAG AGT      304
   Lys Glu Thr Leu Thr Leu Cys Arg Ala Asp Leu Leu Asp Phe Gln Ser
    55                  60                  65                  70
   TTG CGA GAA GCA ATC AGC GGC TGT GAC GGA GTT TTC CAC ACA CGT TCT      352
   Leu Arg Glu Ala Ile Ser Gly Cys Asp Gly Val Phe His Thr Arg Ser
                    75                  80                  85
   CCT GTC ACT GAT GAT CCA GAA CAA ATG GTG GAG CCA GCA GTT ATT GGT      400
   Pro Val Thr Asp Asp Pro Glu Gln Met Val Glu Pro Ala Val Ile Gly
                90                  95                 100
   ACA AAG AAT GTG ATA ACG GCA GCA GCA GAG GCC AAG GTG CGA CGT GTG      448
   Thr Lys Asn Val Ile Thr Ala Ala Ala Glu Ala Lys Val Arg Arg Val
           105                 110                 115
   GTG TTC ACT TCG TCA ATT GGT GCT GTG TAT ATG GAC CCA AAC AGG GAC      496
   Val Phe Thr Ser Ser Ile Gly Ala Val Tyr Met Asp Pro Asn Arg Asp
       120                 125                 130
   CCT GAT AAG GTT GTC GAC GAG ACT TGT TGG AGT GAT CCT GAC TTC TGC      544
   Pro Asp Lys Val Val Asp Glu Thr Cys Trp Ser Asp Pro Asp Phe Cys
   135                 140                 145                 150
   AAA AAC ACC AAG AAT TGG TAT TGT TAT GGG AAG ATG GTG GCA GAA CAA      592
   Lys Asn Thr Lys Asn Trp Tyr Cys Tyr Gly Lys Met Val Ala Glu Gln
                   155                 160                 165
   GCA GCA TGG GAC GAA GCA AGG GAG AAA GGA GTC GAT TTG GTG GCA ATC      640
   Ala Ala Trp Asp Glu Ala Arg Glu Lys Gly Val Asp Leu Val Ala Ile
               170                 175                 180
   AAC CCA GTG TTG GTG CTT GGA CCA CTG CTC CAA CAG AAT GTG AAT GCC      688
   Asn Pro Val Leu Val Leu Gly Pro Leu Leu Gln Gln Asn Val Asn Ala
           185                 190                 195
   AGT GTT CTT CAC ATC CAC AAG TAC CTA ACT GGC TCT GCT AAA ACA TAT      736
   Ser Val Leu His Ile His Lys Tyr Leu Thr Gly Ser Ala Lys Thr Tyr
       200                 205                 210
   ACG TCC AAT TCA CTT CAG GCA TAT GTT CAT GTT AGG GAT GTG GCT TTA      784
   Thr Ser Asn Ser Leu Gln Ala Tyr Val His Val Arg Asp Val Ala Leu
   215                 220                 225                 230
   CGT CAC ATA CTT GTG TAC GAG ACA CCT TCT GCA TCT GGC CGT TAT CTC      832
   Arg His Ile Leu Val Tyr Glu Thr Pro Ser Ala Ser Gly Arg Tyr Leu
                   235                 240                 245
   TGT GCC GAG AGT GTG CTG CAT CGC TGC GAT GTG GTT GAA ATT CTC GCC      880
   Cys Ala Glu Ser Val Leu His Arg Cys Asp Val Val Glu Ile Leu Ala
               250                 255                 260
   AAA TTC TTC CCG GAG TAT CCT ATC CCC ACC AAG TGT TCA GAT GTG ACG      928
   Lys Phe Phe Pro Glu Tyr Pro Ile Pro Thr Lys Cys Ser Asp Val Thr
           265                 270                 275
   AAG CCA AGG GTA AAA CCG TAC AAA TTC TCA AAC CAA AAG CTA AAG GAT      976
   Lys Pro Arg Val Lys Pro Tyr Lys Phe Ser Asn Gln Lys Leu Lys Asp
       280                 285                 290
   TTG GGT CTG GAG TTT ACA CCA GTA CAA TGC TTA TAT GAA ACG GTG AAG     1024
   Leu Gly Leu Glu Phe Thr Pro Val Gln Cys Leu Tyr Glu Thr Val Lys
   295                 300                 305                 310
   AGT CTA CAA GAG AAA GGT CAC CTT CCA ATT CCT ACT CAA AAG GAT GAG     1072
   Ser Leu Gln Glu Lys Gly His Leu Pro Ile Pro Thr Gln Lys Asp Glu
                   315                 320                 325
   ATT ATT CGA ATT CAG TCT GAG AAA TTC AGA AGC TCT TAGCATGTAT          1118
   Ile Ile Arg Ile Gln Ser Glu Lys Phe Arg Ser Ser
               330                 335
   TGAGGAAAAG GGATCAATGG TTAAAGTTGA CCATGGCGTT GTCCCTTTAT GTACCAAGAC   1178
   CAAATGCACC TAGAAATTTA CTTGTCTACT CTGTTGTACT TTTACTTGTC ATGGAAATGT   1238
   TTTTAGTGTT TTCATTGTTA TGAGATATAT TTTGGTGTAA AAAAAAAAAA AAAAA 1293
(2) INFORMATION POUR LA SEQ ID NO: 12:
   (i) CARACTERISTIQUES DE LA SEQUENCE:
      (A) LONGUEUR: 338 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 12:
   Met Pro Ser Val Ser Gly Gln Ile Val Cys Val Thr Gly Ala Gly Gly
    1               5                  10                  15
   Phe Ile Ala Ser Trp Leu Val Lys Ile Leu Leu Glu Lys Gly Tyr Thr
               20                  25                  30
   Val Arg Gly Thr Val Arg Asn Pro Asp Asp Arg Lys Asn Ser His Leu
           35                  40                  45
   Arg Glu Leu Glu Arg Ala Lys Glu Thr Leu Thr Leu Cys Arg Ala Asp
       50                  55                  60
   Leu Leu Asp Phe Gln Ser Leu Arg Glu Ala Ile Ser Gly Cys Asp Gly
   65                  70                  75                  80
   Val Phe His Thr Arg Ser Pro Val Thr Asp Asp Pro Glu Gln Met Val
                   85                  90                  95
   Glu Pro Ala Val Ile Gly Thr Lys Asn Val Ile Thr Ala Ala Ala Glu
               100                 105                 110
   Ala Lys Val Arg Arg Val Val Phe Thr Ser Ser Ile Gly Ala Val Tyr
           115                 120                 125
   Met Asp Pro Asn Arg Asp Pro Asp Lys Val Val Asp Glu Thr Cys Trp
   130 135 140
   Ser Asp Pro Asp Phe Cys Lys Asn Thr Lys Asn Trp Tyr Cys Tyr Gly
   145 150 155 160
   Lys Met Val Ala Glu Gln Ala Ala Trp Asp Glu Ala Arg Glu Lys Gly
   165 170 175
   Val Asp Leu Val Ala Ile Asn Pro Val Leu Val Leu Gly Pro Leu Leu
   180 185 190
   Gln Gln Asn Val Asn Ala Ser Val Leu His Ile His Lys Tyr Leu Thr
   195 200 205
   Gly Ser Ala Lys Thr Tyr Thr Ser Asn Ser Leu Gln Ala Tyr Val His
   210 215 220
   Val Arg Asp Val Ala Leu Arg His Ile Leu Val Tyr Glu Thr Pro Ser
   225 230 235 240
   Ala Ser Gly Arg Tyr Leu Cys Ala Glu Ser Val Leu His Arg Cys Asp
   245 250 255
   Val Val Glu Ile Leu Ala Lys Phe Phe Pro Glu Tyr Pro Ile Pro Thr
   260 265 270
   Lys Cys Ser Asp Val Thr Lys Pro Arg Val Lys Pro Tyr Lys Phe Ser
   275 280 285
   Asn Gln Lys Leu Lys Asp Leu Gly Leu Glu Phe Thr Pro Val Gln Cys
   290 295 300
   Leu Tyr Glu Thr Val Lys Ser Leu Gln Glu Lys Gly His Leu Pro Ile
   305 310 315 320
   Pro Thr Gln Lys Asp Glu Ile Ile Arg Ile Gln Ser Glu Lys Phe Arg
   325 330 335
   Ser Ser
(2) INFORMATION POUR LA SEQ ID NO: 13:
   (i) CARACTERISTIQUES DE LA SEQUENCE:
      (A) LONGUEUR: 1297 paires de bases
      (B) TYPE: acide nucléique
      (C) NOMBRE DE BRINS: double
      (D) CONFIGURATION: linéaire
   (ii) TYPE DE MOLECULE: ADNc pour ARNm
   (ix) CARACTERISTIQUE ADDITIONELLE:
      (A) NOM/CLE: CDS
      (B) EMPLACEMENT: 136..1140
   (xi) DESCRIPTION DE LA SEQUENCE: SEQ ID NO: 13:
   CGGCCGGGAC GACCCGTTCC TCTTCTTCCG GGTCACCGTC ACCATGTTAC ACAACATCTC      60
   CGGCTAAAAA AAAAAGGAAA AAAAGCGCAA CCTCCACCTC CTGAACCCCT CTCCCCCCTC     120
   GCCGGCAATC CCACC ATG CCC GTC GAC GCC CTC CCC GGT TCC GGC CAG ACC      171
                    Met Pro Val Asp Ala Leu Pro Gly Ser Gly Gln Thr
                      1               5                  10
   GTC TGC GTC ACC GGC GCC GGC GGG TTC ATC GCC TCC TGG ATT GTC AAG       219
   Val Cys Val Thr Gly Ala Gly Gly Phe Ile Ala Ser Trp Ile Val Lys
            15                  20                  25
   CTT CTC CTC GAG CGA GGC TAC ACC GTG CGA GGA ACC GTC AGG AAC CCA       267
   Leu Leu Leu Glu Arg Gly Tyr Thr Val Arg Gly Thr Val Arg Asn Pro
        30                  35                  40
   GAC GAC CCG AAG AAT GGT CAT CTG AGA GAT CTG GAA GGA GCC AGC GAG       315
   Asp Asp Pro Lys Asn Gly His Leu Arg Asp Leu Glu Gly Ala Ser Glu
    45                  50                  55                  60
   AGG CTG ACG CTG TAC AAG GGT GAT CTG ATG GAC GAC GGG AGC TTG GAA       363
   Arg Leu Thr Leu Tyr Lys Gly Asp Leu Met Asp Asp Gly Ser Leu Glu
                    65                  70                  75
   GAA GCC ATC AAG GGG TGC GAC GGC GTC GTC CAC ACC GCC TCT CCG GTC 411
   Glu Ala Ile Lys Gly Cys Asp Gly Val Val His Thr Ala Ser Pro Val
   80 85 90
   ACC GAC GAT CCT GAG CAA ATG GTG GAG CCA GCG GTG ATC GGG ACG AAA 459
   Thr Asp Asp Pro Glu Gln Met Val Glu Pro Ala Val Ile Gly Thr Lys
   95 100 105
   AAT GTG ATC GTC GCA GCG GCG GAG GCC AAG GTC CGG CGG GTT GTG TTC 507
   Asn Val Ile Val Ala Ala Ala Glu Ala Lys Val Arg Arg Val Val Phe
   110 115 120
   ACC TCC TCC ATC GGT GCA GTC ACC ATG GAC CCC AAC CGG GCA GAC GTT 555
   Thr Ser Ser Ile Gly Ala Val Thr Met Asp Pro Asn Arg Ala Asp Val
   125 130 135 140
   GTG GTG GAC GAG TCT TGT TGG AGC GAC CTC GAA TTT TGC AAG AGC ACT 603
   Val Val Asp Glu Ser Cys Trp Ser Asp Leu Glu Phe Cys Lys Ser Thr
   145 150 155
   AAG AAC TGG TAT TGC TAC GGC AAG GCA GTG GCG GAG AAG GCC GCT TGG 651
   Lys Asn Trp Tyr Cys Tyr Gly Lys Ala Val Ala Glu Lys Ala Ala Trp
   160 165 170
   CCA GAG GGC AAG GAG AGA GGG GTT GAC CTC GTG GTG ATT AAC CCT GTG 699
   Pro Glu Gly Lys Glu Arg Gly Val Asp Leu Val Val Ile Asn Pro Val
   175 180 185
   CTC GTG CTT GGA CCG CTC CTT CAG TCG ACG ATC AAT GCG AGC ATC ATC 747
   Leu Val Leu Gly Pro Leu Leu Gln Ser Thr Ile Asn Ala Ser Ile Ile
   190 195 200
   CAC ATC CTC AAG TAC TTG ACT GGC TCA GCC AAG ACC TAC GCC AAC TCG 795
   His Ile Leu Lys Tyr Leu Thr Gly Ser Ala Lys Thr Tyr Ala Asn Ser
   205 210 215 220
   GTC CAG GCG TAC GTG CAC GTC AAG GAC GTC GCG CTT GCC CAC GTC CTT 843
   Val Gln Ala Tyr Val His Val Lys Asp Val Ala Leu Ala His Val Leu
   225 230 235
   GTC TTG GAG ACC CCA TCC GCC TCA GGC CGC TAT TTG TGC GCC GAG AGC      891
   Val Leu Glu Thr Pro Ser Ala Ser Gly Arg Tyr Leu Cys Ala Glu Ser
               240                 245                 250
   GTC CTC CAC CGT GGC GAT GTG GTG GAA ATC CTT GCC AAG TTC TTC CCT      939
   Val Leu His Arg Gly Asp Val Val Glu Ile Leu Ala Lys Phe Phe Pro
           255                 260                 265
   GAG TAT AAT GTA CCG ACC AAG TGC TCT GAT GAG GTG AAC CCA AGA GTA      987
   Glu Tyr Asn Val Pro Thr Lys Cys Ser Asp Glu Val Asn Pro Arg Val
       270                 275                 280
   AAA CCA TAC AAG TTC TCC AAC CAG AAG CTG AGA GAC TTG GGG CTC GAG     1035
   Lys Pro Tyr Lys Phe Ser Asn Gln Lys Leu Arg Asp Leu Gly Leu Glu
   285                 290                 295                 300
   TTC ACC CCG GTG AAG CAG TGC CTG TAC GAA ACT GTC AAG AGC TTG CAG     1083
   Phe Thr Pro Val Lys Gln Cys Leu Tyr Glu Thr Val Lys Ser Leu Gln
                   305                 310                 315
   GAG AAA GGC CAC CTA CCA GTC CCC TCC CCG CCG GAA GAT TCG GTG CGT     1131
   Glu Lys Gly His Leu Pro Val Pro Ser Pro Pro Glu Asp Ser Val Arg
               320                 325                 330
   ATT CAG GGA TGATCTTAGA TCCATCACGG TGCGCATTTG TAATCCGGAG             1180
   Ile Gln Gly
           335
   AAATGAGAGA AACATGTGGG AATTTGTTTG TACTTTTCTA AGTCAAACCT GGAGATACCA   1240
   ACCCTGAGTT CTGCATTGGA ATGGAAGTTG TCAATTGTTC CAAAAAAAAA AAAAAAA      1297
(2) INFORMATION POUR LA SEQ ID NO: 14:
   (i) CARACTERISTIQUES DE LA SEQUENCE:
      (A) LONGUEUR: 335 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 14:
   Met Pro Val Asp Ala Leu Pro Gly Ser Gly Gln Thr Val Cys Val Thr
   1 5 10 15
   Gly Ala Gly Gly Phe Ile Ala Ser Trp Ile Val Lys Leu Leu Leu Glu
   20 25 30
   Arg Gly Tyr Thr Val Arg Gly Thr Val Arg Asn Pro Asp Asp Pro Lys
   35 40 45
   Asn Gly His Leu Arg Asp Leu Glu Gly Ala Ser Glu Arg Leu Thr Leu
   50 55 60
   Tyr Lys Gly Asp Leu Met Asp Asp Gly Ser Leu Glu Glu Ala Ile Lys
   65 70 75 80
   Gly Cys Asp Gly Val Val His Thr Ala Ser Pro Val Thr Asp Asp Pro
   85 90 95
   Glu Gln Met Val Glu Pro Ala Val Ile Gly Thr Lys Asn Val Ile Val
   100 105 110
   Ala Ala Ala Glu Ala Lys Val Arg Arg Val Val Phe Thr Ser Ser Ile
   115 120 125
   Gly Ala Val Thr Met Asp Pro Asn Arg Ala Asp Val Val Val Asp Glu
   130 135 140
   Ser Cys Trp Ser Asp Leu Glu Phe Cys Lys Ser Thr Lys Asn Trp Tyr
   145 150 155 160
   Cys Tyr Gly Lys Ala Val Ala Glu Lys Ala Ala Trp Pro Glu Gly Lys
   165 170 175
   Glu Arg Gly Val Asp Leu Val Val Ile Asn Pro Val Leu Val Leu Gly
   180 185 190
   Pro Leu Leu Gln Ser Thr Ile Asn Ala Ser Ile Ile His Ile Leu Lys
   195 200 205
   Tyr Leu Thr Gly Ser Ala Lys Thr Tyr Ala Asn Ser Val Gln Ala Tyr
   210 215 220
   Val His Val Lys Asp Val Ala Leu Ala His Val Leu Val Leu Glu Thr
   225 230 235 240
   Pro Ser Ala Ser Gly Arg Tyr Leu Cys Ala Glu Ser Val Leu His Arg
   245 250 255
   Gly Asp Val Val Glu Ile Leu Ala Lys Phe Phe Pro Glu Tyr Asn Val
   260 265 270
   Pro Thr Lys Cys Ser Asp Glu Val Asn Pro Arg Val Lys Pro Tyr Lys
   275 280 285
   Phe Ser Asn Gln Lys Leu Arg Asp Leu Gly Leu Glu Phe Thr Pro Val
   290 295 300
   Lys Gln Cys Leu Tyr Glu Thr Val Lys Ser Leu Gln Glu Lys Gly His
   305 310 315 320
   Leu Pro Val Pro Ser Pro Pro Glu Asp Ser Val Arg Ile Gln Gly
   325 330 335